(19) Europäisches Patentamt
European Patent Office
Office européen des brevets

(11) **EP 4 715 042 A1**

(12) **EUROPEAN PATENT APPLICATION**
published in accordance with Art. 153(4) EPC

(43) Date of publication:
**25.03.2026 Bulletin 2026/13**

(21) Application number: **24807174.8**

(22) Date of filing: **13.05.2024**

(51) International Patent Classification (IPC):
*C12N 5/071* (2010.01)    *C12M 3/00* (2006.01)
*C12N 5/07* (2010.01)

(52) Cooperative Patent Classification (CPC):
**C12M 3/00; C12N 5/06**

(86) International application number:
**PCT/JP2024/017570**

(87) International publication number:
**WO 2024/237222 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.05.2023 JP 2023080024
15.05.2023 JP 2023080030**

(71) Applicant: **Kuraray Co., Ltd.
Kurashiki-shi, Okayama 710-0801 (JP)**

(72) Inventors:
• **AYANO, Satoru
Tsukuba-shi, Ibaraki 305-0841 (JP)**
• **FUJITA, Akio
Tsukuba-shi, Ibaraki 305-0841 (JP)**
• **KOBAYASHI, Goro
Tokyo 100-0004 (JP)**
• **HIRAI, Yusuke
Tsukuba-shi, Ibaraki 305-0841 (JP)**

(74) Representative: **Müller-Boré & Partner
Patentanwälte PartG mbB
Friedenheimer Brücke 21
80639 München (DE)**

(54) **METHOD FOR PRODUCING CELLS**

(57)    The present invention relates to a method for producing cells, comprising: a step of preparing a culture vessel comprising a composition comprising a liquid medium, a hydrogel containing a crosslinked body of a vinyl alcohol-based polymer, and raw material cells; a step of culturing the raw material cells on a surface of the hydrogel; and a step of detaching the cultured cells from the surface of the hydrogel by a liquid flow.

EP 4 715 042 A1

**Description**

TECHNICAL FIELD

The present patent application claims the benefit of priority under the Paris Convention based on Japanese Patent Application No. 2023-80024 (filed on May 15, 2023) and Japanese Patent Application No. 2023-80030 (filed on May 15, 2023), the entire disclosures of which are hereby incorporated herein by reference.
The present invention relates to a method for producing cells using a hydrogel.

BACKGROUND ART

In the field of regenerative medicine or cancer treatment, cell therapy is being performed in which cells having specific functions are produced ex vivo and these cells are administered to a patient to restore functions of the human body lost due to disease, accident, or other causes. Many of these therapeutic cells are adherent cells that require a scaffold for survival, proliferation, and functional expression.
In conventional production of adherent cells, generally, when cells proliferate through culturing and cover the surface of the cell culture carrier to reach a so-called confluent state, the cells adhering to the surface of the cell culture carrier are detached using a proteolytic enzyme such as trypsin, and the detached cells are either recovered or re-adhered to the surface of a new cell culture carrier and used as raw material cells. However, there is a problem in that such proteolytic enzymes may cause damage to the target cells. In addition, since the proteolytic enzymes are usually derived from an animal, there may be a risk that foreign substances such as viruses from such animals may contaminate the culture system via the proteolytic enzymes. Therefore, methods for detaching cells without using proteolytic enzymes have been studied to date.
For example, Patent Document 1 discloses a method for harvesting cultured cells from a soluble substrate, the method comprising: a step of separating the cultured cells from the substrate by digesting the substrate through exposure to (i) a chelating agent, (ii) an enzyme, or (iii) a chelating agent and an enzyme, the separating resulting in a harvest solution; and a step of performing a series of wash and/or centrifugation cycles of components of the harvest solution following the separation step, wherein the substrate comprises a polygalacturonic acid compound selected from at least one of pectic acid, partially esterified pectic acid, partially amidated pectic acid, and salts thereof, and an adhesive polymer on the surface of the polygalacturonic acid compound.
Patent Document 2 discloses a temperature-sensitive polymer compound obtained by graft-polymerizing N-isopropylacrylamide polymer onto gelatin. For example, it is described that, by applying an aqueous solution of this temperature-sensitive polymer compound to the surface of a culture dish and drying it to obtain a sheet-like cell culture substrate, and after adhering cells on the surface and culturing it, by adjusting the temperature to below the lower critical solution temperature of the temperature-sensitive polymer compound [in polyisopropylacrylamide (PNIPAM)-grafted gelatin, typically 31°C], the surface of the temperature-sensitive polymer compound becomes hydrophilic, thereby reducing cell adhesiveness and enabling the cells to be detached from the cell culture substrate.
Patent Document 3 discloses a method for culturing adherent cells, comprising, culturing the adherent cells in a liquid medium in an alicyclic structure-containing polymer culture vessel, detaching the cells from the culture vessel by liquid flow without adding a proteolytic enzyme, and suspending the cultured cells in a liquid medium.
Patent Document 4 discloses a method for culturing stem cells, comprising, proliferating stem cells adhered to a culture vessel in a culture vessel containing a liquid medium, detaching the proliferated stem cells from the culture vessel by a detachment solution containing at least one chelating agent and not containing a proteolytic enzyme, and suspending the detached stem cells in a liquid medium, wherein at least the part of the culture vessel to which the stem cells adhere is formed of an alicyclic structure-containing polymer.

PRIOR ART DOCUMENT

PATENT DOCUMENT

Patent Document 1: JP-A-2022-536651
Patent Document 2: JP-A-11-349643
Patent Document 3: WO 2017/104618 A1
Patent Document 4: JP-A-2018-201403

SUMMARY OF THE INVENTION

PROBLEMS TO BE SOLVED BY THE INVENTION

According to the investigations by the present inventors, in the method of Patent Document 1, since the components of the dissolved substrate and the digestion reagents are recovered together with the target cells, it is necessary to perform washing to remove these non-target substances. However, because the recovered material is in liquid form, there is a problem in that the non-target substances cannot be completely removed. In the method of Patent Document 2, it is necessary to lower the temperature from the culture temperature (typically approximately 37°C) to the detachment temperature (31°C or lower) in order to detach the cells from the cell culture substrate. However, when this temperature decrease takes time, there is a risk that the activity of the cells may decline during that period. In particular, in scaled-up culture vessels (for example, large-capacity industrial bioreactors), the above-mentioned temperature decrease requires a long time (for example, several hours or more), and therefore, the method described in the above document cannot be substantially adopted for production of cells in scaled-up culture vessels. In addition, Patent Documents 3 and 4 disclose culturing methods using culture vessels made of a polymer containing an alicyclic structure. However, since cells do not spread on polymers containing alicyclic structures, they can only be used for planar culture. For example, in industrial bioreactors where scaffold materials are suspended in a liquid medium, it is difficult to perform culturing, and thus the methods of Patent Documents 3 and 4 cannot be substantially adopted for industrial production of cells.

In view of the above, an object of the present invention is to provide a method for producing cells, wherein cells can be detached from a cell culture carrier without substantially using a proteolytic enzyme or using it only at a low concentration, thereby enabling the stable production of cells with well-maintained activity.

SOLUTIONS TO THE PROBLEMS

The present inventors conducted extensive studies on methods for producing cells in order to solve the above problem, and as a result, completed the present invention.

That is, the present invention includes the following preferred embodiments.

[1] A method for producing cells, comprising:

a step of preparing a culture vessel comprising a composition comprising a liquid medium, a hydrogel containing a crosslinked body of a vinyl alcohol-based polymer, and raw material cells;
a step of culturing the raw material cells on a surface of the hydrogel; and
a step of detaching the cultured cells from the surface of the hydrogel by a liquid flow.

[2] A method for producing cells, comprising:

a step of preparing a culture vessel comprising a composition comprising a liquid medium, a hydrogel containing a crosslinked body of a vinyl alcohol-based polymer, and raw material cells;
a step of culturing the raw material cells on a surface of the hydrogel; and
a step of detaching the cultured cells from the surface of the hydrogel by bringing at least a part of the composition in the culture vessel into contact with a solution in which a concentration of a proteolytic enzyme is 1.5 mg/mL or less and a concentration of a chelating agent is 0.01 mg/mL or more.

[3] The method according to [2], further comprising:
a step of detaching the cultured cells from the surface of the hydrogel by a liquid flow.
[4] The method according to [1] or [3], wherein
the liquid flow is generated by one or more means selected from the group consisting of the following (i) to (iv):

(i) suction and discharge of at least a part of the composition through a tube installed in the culture vessel;
(ii) shaking of the composition;
(iii) stirring of the composition by stirring blades; and
(iv) discharge of a gas and/or a liquid into the culture vessel.

[5] The method according to any one of [1] to [4], wherein the hydrogel comprises a cell adhesion factor.
[6] The method according to any one of [1] to [5], wherein the hydrogel is non-spherical particles, spherical particles, a fine molded article, an article of any shape formed by a 3D printer, a film, filament, hollow fibers, a porous monolith, a coated article, or a mixture of two or more thereof.
[7] The method according to [6], wherein the hydrogel in an equilibrium swollen state in a liquid medium is spherical particles having an average particle diameter of 10 to 5,000 μm.

[8] The method according to any one of [1] to [7], wherein the vinyl alcohol-based polymer has an ethylenically unsaturated group and has a viscosity-average degree of polymerization of 300 or more as measured in accordance with JIS K 6726:1994.

[9] The method according to any one of [2] to [8], wherein the chelating agent is ethylenediaminetetraacetic acid or ethylene glycol tetraacetic acid.

[10] The method according to any one of [1] to [9], wherein the culture vessel is a bioreactor.

[11] The method according to any one of [1] to [10], further comprising:

a step of separating the detached cells and the hydrogel.

[12] The method according to [11], wherein the detached cells and the hydrogel are separated using a porous material.

[13] The method according to [11], wherein the detached cells and the hydrogel are separated based on a difference in sedimentation velocity.

[14] A method for producing cells, comprising:

a step of recovering the hydrogel separated by the method according to any one of [11] to [13];
a step of preparing a culture vessel containing a composition comprising a liquid medium, the recovered hydrogel, and raw material cells; and
a step of culturing the raw material cells on a surface of the recovered hydrogel.

EFFECTS OF THE INVENTION

According to the present invention, it is possible to provide a method for producing cells, wherein cells can be detached from a cell culture carrier without substantially using a proteolytic enzyme or by using it only at a low concentration, thereby enabling the stable production of cells with well-maintained activity.

DETAILED DESCRIPTION

Hereinafter, embodiments of the present invention will be described in detail. The scope of the present invention is not limited to the embodiment described herein, and various modifications can be made without impairing the gist of the present invention.

A method for producing cells of the present invention, comprises:

a step of preparing a culture vessel comprising a composition comprising a liquid medium, a hydrogel containing a crosslinked body of a vinyl alcohol-based polymer, and raw material cells (preparation step);
a step of culturing the raw material cells on a surface of the hydrogel (culturing step); and
a step of detaching the cultured cells from the surface of the hydrogel by a liquid flow (detachment step). Hereinafter, this production method is also referred to as a "first production method".

[Preparation Step of First Production Method]

The composition contained in the culture vessel comprises a liquid medium, a hydrogel containing a crosslinked body of a vinyl alcohol-based polymer, raw material cells, and, as needed, optional components. Into the culture vessel, a composition obtained by mixing a liquid medium, a hydrogel, raw material cells, and, as needed, optional components may be introduced, a liquid medium, a hydrogel, raw material cells, and, as needed, optional components may be introduced in any order, or a premix obtained by mixing two or more substances selected from the group consisting of a liquid medium, a hydrogel, raw material cells, and, as needed, optional components in advance and the remaining substances may be introduced in any order. The mixing or introduction method for obtaining the composition or the mixing or introduction method for obtaining the premix is not particularly limited, and a conventional method may be adopted.

The hydrogel before mixing or introduction may be in a dry state or may contain a liquid such as water, a water-soluble organic solvent, or a liquid medium (preferably, the same liquid medium as the liquid medium contained in the composition). In addition, the hydrogel may be in an equilibrium swollen state with the liquid. In the present specification, the dry state refers to a state where volatile components such as water are reduced. For example, by performing drying such as hot air drying, ventilation drying, vacuum drying, or freeze drying until the mass of a substance (for example, a hydrogel) reaches a constant weight, the substance can be brought into a dry state. The equilibrium swollen state means a state of swelling with a liquid until equilibrium is reached.

By immersing the hydrogel in the liquid, it is possible to remove unreacted raw materials containing a radical polymerization initiator, non-crosslinked polymer components, and/or other residues. This allows for a further reduction in the content of the unreacted raw materials, the non-crosslinked polymer components, and/or other residues, resulting in a decrease in the toxicity of the hydrogel to cells. When it is desired to further remove the unreacted raw materials, the non-crosslinked polymer components, and/or other residues, the immersion operation may be repeated by replacing the liquid. In addition, the hydrogel may be mixed or introduced after being brought into a dry state and then immersed again in the liquid to be brought into an equilibrium swollen state. The hydrogel can be dried, for example, by undergoing any drying step described later.

The preparation step is usually performed under the same temperature and atmosphere conditions as in a culturing step described later.

<Hydrogel>

In the culture vessel in the preparation step, the raw material cells are present on the surface of the hydrogel containing a crosslinked body of a vinyl alcohol-based polymer. In the subsequent culturing step, the raw material cells proliferate using the hydrogel as a scaffold. That is, the hydrogel serves as a cell culture carrier.

The hydrogel contains a crosslinked body of a vinyl alcohol-based polymer and, as needed, optional components other than cell adhesion factors, and preferably contains a crosslinked body of a vinyl alcohol-based polymer, a cell adhesion factor, and, as needed, the optional components.

The degree of swelling of the hydrogel represented by the following Equation (1) is preferably 200% or more, more preferably 300% or more, and still more preferably 400% or more. When the degree of swelling is equal to or more than the lower limit, the hydrogel can swell in the liquid medium even with a relatively small proportion of the dry mass of the hydrogel, thereby providing a sufficient scaffold. The degree of swelling can be controlled to be equal to or greater than the aforementioned lower limit, for example, by adjusting the amount of the polyfunctional crosslinking agent in the crosslinked body of the vinyl alcohol-based polymer, adjusting the amount of the ethylenically unsaturated group, and/or adjusting the content of the vinyl alcohol-based polymer in the uncrosslinked polymer solution, in the process of preparing the hydrogel. The upper limit of the degree of swelling is not particularly limited, and is usually 10,000% or less. The degree of swelling can be calculated using the following Equation (1), by first measuring the mass of the hydrogel in its dry state, then immersing it in ion-exchanged water, physiological saline, or phosphate-buffered saline for at least 2 hours, and removing the liquid on the hydrogel surface either by blotting with a paper sheet or by centrifugation, and finally measuring the mass of the hydrogel in its swollen state.

Degree of swelling [%] = (Mass of hydrogel in swollen state [g]/Mass of hydrogel in dry state [g]) x 100     Equation (1)

The hydrogel is preferably non-spherical particles, spherical particles, a fine molded article, an article of any shape formed by a 3D printer, a film, filament, hollow fibers, a porous monolith, a coated article, or a mixture of two or more thereof. The spherical particles include not only perfectly spherical particles but also elliptically spherical particles, partially missing or protruding spherical particles, partially missing or protruding elliptically spherical particles, spherical particles having protrusions or wrinkles on the surface, and porous spheres. The fine molded article refers to a molded article having fine irregularities on surface or inside thereof. The fine processing size when the hydrogel is in an equilibrium swollen state is typically 10 to 1,000 $\mu$m. The shape formed by a 3D printer is, for example, any shape that can be molded by a 3D printer of a stereolithography type, an inkjet type, or a nozzle extrusion type.

In one embodiment, the hydrogel in an equilibrium swollen state in a liquid medium (particularly a liquid medium contained in the composition) is preferably spherical particles having an average particle diameter of preferably 10 $\mu$m or more, more preferably 20 $\mu$m or more, even more preferably 50 $\mu$m or more, still even more preferably 80 $\mu$m or more, and still even more preferably 100 $\mu$m or more, and preferably 5,000 $\mu$m or less, more preferably 4,500 $\mu$m or less, even more preferably 4,000 $\mu$m or less, still even more preferably 3,000 $\mu$m or less, still even more preferably 2,000 $\mu$m or less, and still even more preferably 1,500 $\mu$m or less. The range of the average particle diameter is preferably 10 to 5,000 $\mu$m, more preferably 20 to 4,500 $\mu$m, even more preferably 50 to 4,000 $\mu$m, still even more preferably 80 to 3,000 $\mu$m, still even more preferably 100 to 2,000 $\mu$m, and still even more preferably 100 to 1,500 $\mu$m.

In one embodiment, the hydrogel in an equilibrium swollen state in a phosphate-buffered saline is preferably spherical particles having an average particle diameter of preferably 10 $\mu$m or more, more preferably 20 $\mu$m or more, still more preferably 50 $\mu$m or more, still even more preferably 80 $\mu$m or more, and still even more preferably 100 $\mu$m or more, and preferably 5,000 $\mu$m or less, more preferably 4,500 $\mu$m or less, still more preferably 4,000 $\mu$m or less, still even more preferably 3,000 $\mu$m or less, still even more preferably 2,000 $\mu$m or less, and still even more preferably 1,500 $\mu$m or less. The range of the average particle diameter is preferably 10 to 5,000 $\mu$m, more preferably 20 to 4,500 $\mu$m, still more preferably 50 to 4,000 $\mu$m, still even more preferably 80 to 3,000 $\mu$m, still even more preferably 100 to 2,000 $\mu$m, and still even more preferably 100 to 1,500 $\mu$m.

The average particle diameter of the hydrogel can be measured using a laser diffraction/scattering type particle diameter distribution measuring apparatus as described in Examples. The average particle diameter in the present invention is a volume-based average particle diameter of a particle diameter (equivalent spherical diameter) in an equilibrium swollen state. Therefore, even when the average particle diameter is measured using a microscope, the average particle diameter can be measured by swelling as described in Examples and then observing the swelling. When the hydrogel is swollen in a liquid (for example, a liquid medium) other than phosphate-buffered saline, the hydrogel is washed with water and dried by a general method, and then brought into an equilibrium swollen state with phosphate-buffered saline, whereby the average particle diameter of the hydrogel in the equilibrium swollen state with phosphate-buffered saline can also be measured. It is preferable that the average particle diameter is equal to or greater than the lower limit and equal to or less than the upper limit, or within the above range, because such a size provides excellent handling property and allows for easy and rapid separation of the detached cells from the hydrogel in the separation step described later. The easy and rapid separation not only improves the efficiency of cell production, but also better maintains the activity of the produced cells, thereby enabling more stable cell production. This advantage is also useful when the culture vessel is a large volume bioreactor. The average particle diameter can be controlled to be equal to or greater than the above lower limit and equal to or less than the upper limit, or within the above range, for example, by adopting an appropriate molding method and/or selecting a classification method after molding in the process of preparing the hydrogel.

Other than spherical particles, the hydrogel is also preferably a film, filament, or a coated article.

In one embodiment in which the hydrogel is in the form of a film, for example, when the film-shaped hydrogel is used in a fluidized bed type bioreactor or a stirred tank type bioreactor described later, the film-shaped hydrogel needs to be dispersed in the composition. Therefore, the thickness, width, and length of the film-shaped hydrogel that is brought into an equilibrium swollen state by the liquid medium (particularly, the liquid medium contained in the composition) are each preferably 10 to 5,000 $\mu$m, more preferably 30 to 2,000 $\mu$m, and still more preferably 50 to 1,000 $\mu$m.

On the other hand, when the film-shaped hydrogel is used in a fixed bed type bioreactor described later, this is not the case. A larger film-shaped hydrogel may be used, for example, in the form of a spiral membrane. From the viewpoint of retaining the mechanical strength of the film-shaped hydrogel, the film-shaped hydrogel in this case has a thickness of preferably 10 to 5,000 $\mu$m, more preferably 30 to 2,000 $\mu$m, and still more preferably 50 to 1,000 $\mu$m, a width of preferably 10 to 5,000 mm, more preferably 100 to 3,000 mm, and still more preferably 300 to 2,000 mm, and a length of preferably 0.5 to 100 m, more preferably 1 to 50 m, and still more preferably 2 to 30 m. The thickness, width, and length are dimensions for a film-shaped hydrogel that is brought into an equilibrium swollen state by a liquid medium (in particular, a liquid medium contained in the composition).

The thickness, width and length of the film-shaped hydrogel can be measured by observing the film-shaped hydrogel with a microscope or the like.

In one embodiment in which the hydrogel is in the form of filament, for example, when the fibrous hydrogel is used in a fluidized bed type bioreactor or a stirred tank type bioreactor to be described later, the fibrous hydrogel needs to be dispersed in the composition. Therefore, the diameter and length of the fibrous hydrogel that is brought into an equilibrium swollen state by the liquid medium (particularly, the liquid medium contained in the composition) are each preferably 10 to 5,000 $\mu$m, more preferably 30 to 2,000 $\mu$m, and still more preferably 50 to 1,000 $\mu$m. The diameter and length of the fibrous hydrogel may be measured by observing the fibrous hydrogel with a microscope or the like.

The coated article refers to one in which a hydrogel is coated on a substrate [for example, the above-mentioned non-spherical particles, spherical particles, fine molded article, article of any shape formed by a 3D printer, film, tray, filament, hollow fiber, porous monolith, plate to be described later that is commonly used as a culture vessel, well plate, flask (including T-flask, shaker flask, and spinner flask), chamber slide, dish, tube, bottle, roller bottle, and bag (including shaking bag)]. The material of the substrate may be freely selected from, for example, glass, polyolefin, polymethyl methacrylate, polystyrene, polyester, polyolefin, ethylene vinyl alcohol copolymer, polyamide, polyimide, and a combination of two or more thereof. From the viewpoint of preventing peeling of the coated hydrogel from the substrate when the coated hydrogel swells in the liquid medium, the thickness of the hydrogel layer in the coated article that is brought into an equilibrium swollen state in the liquid medium (in particular, the liquid medium contained in the composition) is preferably 0.05 to 1,000 $\mu$m, more preferably 0.1 to 700 $\mu$m, and still more preferably 0.2 to 500 $\mu$m. The thickness of the hydrogel layer may be measured by, for example, a method in which the thickness of the hydrogel layer observed in a cross-sectional image taken by an electron microscope of the coated article in a dry state is converted into the thickness in an equilibrium swollen state; a method in which a part of the hydrogel layer is detached off and the thickness is measured using a film thickness step meter; or a method using an ellipsometer.

The hydrogel is preferably sterilized from the viewpoint of use in the production of cells. The sterilization in the present invention does not necessarily mean sterilization with a sterility assurance level of SAL = $10^{-6}$ or less as required for pharmaceutical products or medical devices used for a human body. The sterility assurance level of the test and research application should be achieved, and is preferably SAL = $10^{-3}$ or less, more preferably SAL = $10^{-4}$ or less, and still more preferably SAL = $10^{-5}$ or less. However, SAL is preferably $10^{-6}$ or less when the hydrogel is incorporated into a part of a pharmaceutical product or a medical device used for a human body, or the hydrogel is used in the manufacture of the article.

The proportion of the hydrogel in the composition is, for example, 0.005 to 50 mass%, preferably 0.01 to 40 mass%, and more preferably 0.05 to 30 mass%, based on the total mass of the composition. In this content, the mass of the hydrogel refers to the mass in its dry state.

<Method for Preparing Hydrogel>

The method for preparing the hydrogel is not particularly limited. The hydrogel can be prepared, for example, by a method comprising the steps of: preparing an uncrosslinked polymer solution containing a vinyl alcohol-based polymer (uncrosslinked polymer solution preparation step); subsequently molding the uncrosslinked polymer solution (molding step); and then crosslinking the molded vinyl alcohol-based polymer (crosslinking step).

(uncrosslinked polymer solution preparation step) First, a vinyl alcohol-based polymer and a method for preparing the same will be described.

The vinyl alcohol-based polymer is not particularly limited as long as it contains more than 50 mol% of structural units derived from vinyl alcohol based on the total structural units constituting the vinyl alcohol-based polymer, and it may contain structural units derived from a vinyl ester. The total amount of the structural units derived from vinyl alcohol and the structural units derived from vinyl ester with respect to the total structural units constituting the vinyl alcohol-based polymer is preferably 80 mol% or more, more preferably 90 mol% or more, and still more preferably 95 mol% or more.

Examples of the method for preparing a vinyl alcohol-based polymer include a method in which a polyvinyl ester obtained by polymerizing a vinyl ester-based monomer is saponified to convert an ester group in the polyvinyl ester into a hydroxyl group.

Examples of the vinyl ester-based monomer include aliphatic vinyl esters such as vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, and vinyl oleate; and aromatic vinyl esters such as vinyl benzoate. These compounds may be used alone or in combination of two or more thereof.

Among the vinyl ester-based monomers, an aliphatic vinyl ester is preferable, and vinyl acetate is more preferable from the viewpoint of preparation cost. That is, the polyvinyl ester is preferably polyvinyl acetate obtained by polymerizing vinyl acetate.

The polyvinyl ester may contain a structural unit derived from a monomer other than the vinyl ester-based monomer as necessary as long as the effect of the present invention is not impaired. Examples of such monomers include: $\alpha$-olefins such as ethylene, propylene, n-butene, and isobutylene; acrylic acid or salts thereof; alkyl acrylates such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylates such as methyl methacrylate, ethyl methacrylate, n-propyl methacrylate, i-propyl methacrylate, n-butyl methacrylate, i-butyl methacrylate, t-butyl methacrylate, 2-ethylhexyl methacrylate, dodecyl methacrylate, and octadecyl methacrylate; acrylamide derivatives such as acrylamide, N-methylacrylamide, N-ethylacrylamide, N,N-dimethylacrylamide, diacetone acrylamide, acrylamidopropanesulfonic acid or salts thereof, acrylamidopropyldimethylamine or salts or quaternary salts thereof, N-methylolacrylamide or derivatives thereof; methacrylamide derivatives such as methacrylamide, N-methylmethacrylamide, N-ethylmethacrylamide, methacrylamidopropanesulfonic acid or salts thereof, methacrylamidopropyldimethylamine or salts or quaternary salts thereof, N-methylolmethacrylamide or derivatives thereof; N-vinylamide derivatives such as N-vinylformamide and N-vinylacetamide; vinyl ethers such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles such as acrylonitrile and methacrylonitrile; halogenated vinyl compounds such as vinyl chloride and vinyl fluoride; halogenated vinylidene compounds such as vinylidene chloride and vinylidene fluoride; allyl compounds such as allyl acetate and allyl chloride; maleic acid or salts, esters, or anhydrides thereof; vinylsilyl compounds such as vinyltrimethoxysilane; and isopropenyl acetate. These compounds may be used alone or in combination of two or more thereof.

When the polyvinyl ester contains structural units derived from monomers other than vinyl ester-based monomers, the content of such structural units is preferably 20 mol% or less, more preferably 10 mol% or less, and still more preferably 5 mol% or less, based on the total structural units constituting the polyvinyl ester.

The method for saponifying the polyvinyl ester is not particularly limited, and the saponification can be performed by a method similar to the conventional method. For example, an alcoholysis method using an alkali catalyst or an acid catalyst, a hydrolysis method may be used. Among them, a saponification reaction using methanol as a solvent and caustic soda (NaOH) as a catalyst is simple and therefore preferable.

The viscosity-average degree of polymerization of the vinyl alcohol-based polymer measured in accordance with JIS K 6726:1994 is preferably 300 or more, more preferably 450 or more, still more preferably 500 or more, and may be 1,000 or more or 1,500 or more, from the viewpoint of suppressing embrittlement when the crosslinked body of the vinyl alcohol-based polymer swells upon contact with a liquid. In addition, the viscosity-average degree of polymerization is preferably 10,000 or less, more preferably 5,000 or less, still more preferably 3,500 or less, and still even more preferably 2,500 or less, from the viewpoint of suppressing an increase in viscosity of the aqueous solution and improving processability when the crosslinked body of the vinyl alcohol-based polymer is prepared.

As the vinyl alcohol-based polymer, a single type may be used, or two or more types having different viscosity-average degrees of polymerization, degrees of saponification (described later), and/or 4 mass% viscosity (described later) may be used in combination. In that case, it is preferable that the at least one vinyl alcohol-based polymer have a viscosity-average degree of polymerization, a degree of saponification and/or 4 mass% viscosity in a preferable range, and it is more preferable that all vinyl alcohol-based polymers have a viscosity-average degree of polymerization, a degree of saponification and/or 4 mass% viscosity in a preferable range.

The degree of saponification of the vinyl alcohol-based polymer measured in accordance with JIS K 6726:1994 is preferably 50 mol% or more, more preferably 60 mol% or more, and still more preferably 65 mol% or more, from the viewpoint of improving the water solubility of the vinyl alcohol-based polymer. The upper limit of the degree of saponification is not particularly limited, and is, for example, 100 mol%, and preferably 99 mol%.

The viscosity of the vinyl alcohol-based polymer at 4 mass% at 20°C is preferably 0.5 to 110 mPa·s, more preferably 1 to 80 mPa·s, and still more preferably 2 to 60 mPa·s. When the viscosity is within the above range, the ease of preparation of the hydrogel is improved, and the mechanical strength of the hydrogel can be improved. For an aqueous solution containing 4 mass% of a vinyl alcohol-based polymer, the viscosity is measured at a temperature of 20°C using a B-type viscometer (rotation speed: 12rpm) in accordance with the rotational viscometer method of JIS K 6726:1994.

By mixing the vinyl alcohol-based polymer as described above with a solvent, an uncrosslinked polymer solution containing the vinyl alcohol-based polymer can be prepared.

The solvent is preferably one or more solvents selected from the group consisting of water and water-soluble organic solvents. The solvent more preferably contains water, and still more preferably is water. Examples of water-soluble organic solvents include aprotic polar solvents such as dimethylformamide, dimethylacetamide, dimethyl sulfoxide, and N-methylpyrrolidone; monoalcohols such as methanol, ethanol, propanol, and isopropanol; and polyhydric alcohols such as ethylene glycol, diethylene glycol, triethylene glycol, and glycerin.

The content of the solvent in the uncrosslinked polymer solution is preferably 50 mass% or more, more preferably 55 mass% or more, still more preferably 60 mass% or more, and preferably 99.999 mass% or less, and more preferably 99.99 mass% or less, and still more preferably 99.9 mass% or less based on the total mass of the uncrosslinked polymer solution.

When the uncrosslinked polymer solution contains a water-soluble organic solvent, the content thereof is preferably 30 mass% or less, more preferably 20 mass% or less, and still more preferably 10 mass% or less based on the total mass of the uncrosslinked polymer solution.

From the viewpoint of sufficient mechanical strength or dimension of the hydrogel, the content of the vinyl alcohol-based polymer in the uncrosslinked polymer solution is preferably 0.001 mass% or more, more preferably 0.01 mass% or more, and still more preferably 0.1 mass% or more based on the total mass of the uncrosslinked polymer solution. In addition, from the viewpoint of suppressing an increase in viscosity of the uncrosslinked polymer solution and obtaining good moldability, the content of the vinyl alcohol-based polymer in the uncrosslinked polymer solution is preferably 50 mass% or less, more preferably 45 mass% or less, and still more preferably 40 mass% or less based on the total mass of the uncrosslinked polymer solution.

(Molding Step)

The method for molding the uncrosslinked polymer solution is not particularly limited, and the uncrosslinked polymer solution may be molded by a known methods into shapes such as non-spherical particles, spherical particles, fine molded articles, articles of any shape formed by a 3D printer, a film, filament, hollow fibers, a porous monolith, and a coated article.

The method for molding the spherical particles is not particularly limited, and a known technique may be used. Examples thereof include a suspension polymerization method, a membrane emulsification method, a microfluidic method, a nozzle extrusion method, and a spray drying method (spray drying). In addition to these, for example, methods such as submerged electrospray, as described in Biotechnology and Bioengineering, 2012, Vol. 109, pp. 1561-1570, may also be used.

The suspension polymerization method is a method for obtaining spherical particles of a hydrogel by crosslinking an uncrosslinked polymer in a water-in-oil liquid-liquid dispersion system in which an uncrosslinked polymer solution containing a radical polymerization initiator is used as a dispersed phase and a liquid incompatible with the dispersed phase is used as a continuous phase. An emulsification stabilizer may be added to the continuous phase as necessary. The dispersed phase is formed into microdroplets by mechanical energy (generally, a stirring blade), and crosslinking of the uncrosslinked polymer proceeds inside the droplets.

The membrane emulsification method is a method in which an uncrosslinked polymer solution containing a radical polymerization initiator is extruded into the continuous phase through a membrane having a uniform pore diameter to form uniform water-in-oil microdroplets, and the uncrosslinked polymer is crosslinked to obtain spherical particles of a hydrogel. As in the suspension polymerization method, an emulsification stabilizer may be added to the continuous phase as necessary. As the membrane having a uniform pore diameter, a Shirasu Porous Glass (SPG) membrane, or a hydrophilic or hydrophobic membrane having uniform pores manufactured by Micropore Technologies, Ltd. may be used. For example, the basic methods described in Journal of Membrane Science, 2017, Volume 524, pp. 79-86 may be used.

The microfluidic method is a method for obtaining spherical particles of a hydrogel by causing the continuous phase to flow in a micrometer-order microchannel, injecting an uncrosslinked polymer solution containing a radical polymerization initiator, which is a dispersed phase, into the continuous phase by the micrometer-order microchannel in the same manner to form microdroplets, and crosslinking the uncrosslinked polymer. Depending on the method of injecting the dispersed phase into the continuous phase, the device can be classified into types such as terrace-like devices, T-junctions, flow focusing microchannel devices (FFD), and capillary-type devices (parallel flow type, co-flow type, and FFD type). For example, methods described in Chemical Engineering and Technology, 2008, Vol. 31, pp. 1099-1115 are applicable. Also in the microfluidic method, an emulsification stabilizer may be added to the continuous phase as necessary.

As the emulsification stabilizer, water-soluble polymers such as partially saponified polyvinyl alcohol, gelatin, hydroxymethyl cellulose, methyl cellulose, and carboxymethyl cellulose; and surfactants such as sodium dodecylbenzenesulfonate, sorbitan monooleate, dioctyl sulfosuccinate, polyoxyethylene sorbitan monooleate (Tween-80), and polyoxyethylene sorbitan monostearate (Tween-60) may be used.

As the liquid incompatible with the dispersed phase, for example, water.insoluble oil, toluene, hexane, octane (including isooctane), liquid paraffin, edible oil, and dichloroethane are generally used.

The nozzle extrusion method is a method in which an uncrosslinked polymer solution containing a radical polymerization initiator is extruded from a nozzle into air and dropped into the continuous phase to form water-in-oil microdroplets, and the uncrosslinked polymer is crosslinked to obtain spherical particles of a hydrogel. In order to efficiently generate droplets, it is also possible to mechanically cut the uncrosslinked polymer solution, for example, by vibrating the nozzle or using a rotating disk or a rotating nozzle. More specific examples of the nozzle extrusion method include a method described in Chemical Papers, 2008, Vol. 62, pp. 364-374. In the nozzle extrusion method, it is also possible to obtain spherical particles of a hydrogel without using the continuous phase by crosslinking an uncrosslinked polymer while droplets are falling in the air.

In the nozzle extrusion method, for example, it is also possible to form spherical particles by dissolving a substance such as alginic acid in an uncrosslinked polymer solution containing a radical polymerization initiator, and then dropping the solution into a multivalent metal ion solution using the nozzle extrusion method. This is a method using the property that alginic acid is gelled by multivalent metal ions, and the hydrogel in the present invention can be obtained by crosslinking the uncrosslinked polymer by the method to be described later after forming spherical particles.

In the spray drying method, an uncrosslinked polymer solution is atomized through a nozzle, a rotating disk or the like to form microdroplets, and the spherical particles are obtained by drying with heat. Crosslinking of the uncrosslinked polymer can be performed, in the case of using a photoradical polymerization initiator, by irradiating light at the stage when microdroplets are formed, and in the case of using a thermal radical polymerization initiator, by drying the microdroplets with heat. Of course, after obtaining the dried spherical particles, it is possible to obtain the hydrogel of the present invention by inducing crosslinking through light or heat, depending on the type of radical polymerization initiator used.

As a method for molding a hydrogel of spherical particles, any of these particle production methods can be suitably used. From the viewpoint of controlling the average particle diameter of the hydrogel, a suspension polymerization method by a static mixer, a membrane emulsification method, a microfluidic method, a nozzle extrusion method, and a spray drying method are preferable, and a membrane emulsification method, a microfluidic method, and a nozzle extrusion method are more preferable. These methods basically have a feature that the volume of microdroplets can be strictly defined using pores or nozzles, and this contributes to the control of the average particle diameter. Of course, it may also be used as a preferable method to control the average particle diameter of spherical particles obtained by classifying spherical particles having a particle diameter distribution produced by a suspension polymerization method in which microdroplets are formed by a stirring blade. From the viewpoint of hydrogel productivity, a suspension polymerization method is preferable.

The film-shaped hydrogel may be molded by pouring an uncrosslinked polymer solution into a glass plate with a spacer interposed therebetween. As a more industrial method, a film-shaped hydrogel may be molded by subjecting an uncrosslinked polymer solution to an extrusion molding method such as a T-die method, a solution casting method. The solution casting method is a method for molding a film by flowing and spreading an uncrosslinked polymer solution in a smooth casting drum or a stainless belt, in which a film-shaped hydrogel can be obtained by crosslinking the uncrosslinked polymer solution formed into a film, or by crosslinking the uncrosslinked polymer after obtaining a dry film by evaporating a solvent of the uncrosslinked polymer solution formed into a film.

The filamentous hydrogel may be molded by a so-called wet spinning method. That is, the hydrogel of the present invention may be obtained by extruding an uncrosslinked polymer solution through a spinneret (nozzle) into a coagulation bath such as dimethyl sulfoxide, a saturated aqueous sodium sulfate solution or a saturated aqueous ammonium sulfate solution to form a filamentous shape, followed by either crosslinking as is or crosslinking after drying.

Examples of methods for coating a substrate with a hydrogel to form a hydrogel as a coated article, include methods in which an uncrosslinked polymer solution is coated onto a substrate using roll coaters such as gravure coaters, reverse coaters, slot-die coaters, lip coaters, knife coaters, or comma coaters; or using devices such as spin coaters, dip coaters, spray coaters, or inkjet coaters. In the case of coating a spherical particle substrate with a hydrogel, it may be performed by coating the spherical particle substrate with an uncrosslinked polymer solution using, for example, a fluidized bed granulation coater.

The above molding methods may be freely and optimally selected depending on the shape of the target hydrogel.

(Crosslinking Step)

The crosslinking step may be performed while the molded vinyl alcohol-based polymer (hereinafter also referred to as a molded vinyl alcohol-based polymer) still contains the solvent, or after removing the solvent from the molded vinyl alcohol-based polymer. When crosslinking is performed while the molded vinyl alcohol-based polymer still contains the solvent, significant shrinkage may occur during the optional drying step described later, which can make it difficult to form a shape such as a film, filament, hollow fibers, or coatings. In this case, it is preferable to perform the crosslinking after removing the solvent from the molded vinyl alcohol-based polymer by drying. As the drying method, the same methods as those described in the drying step below may be employed. The degree of drying may be appropriately selected depending on the composition or shape of the molded vinyl alcohol-based polymer or the crosslinking method. From the viewpoint of shrinkage in the optional drying step described later, the content of the solvent represented by the following Equation (2) is preferably 50 mass% or less, more preferably 25 mass% or less, and still more preferably 10 mass% or less.

$$\text{Solvent content [\%]} = [(\text{Mass of solvent contained in molded vinyl alcohol-based polymer})/(\text{Mass of molded vinyl alcohol-based polymer in dry state + mass of solvent contained in molded vinyl alcohol-based polymer})] \times 100 \qquad \text{Equation (2)}$$

As a method for measuring the solvent content, the same methods as those described in the drying step below may be employed.

The vinyl alcohol-based polymer may be physically crosslinked or may be crosslinked by a covalent bond. From the viewpoint of controlling the physical properties of the obtained hydrogel and/or improving the stability of the hydrogel during swelling, it is preferable to crosslink the hydrogel by a covalent bond.

Examples of the method for physically crosslinking the vinyl alcohol-based polymer include a method for freezing and thawing an aqueous solution of the vinyl alcohol-based polymer; and a method for heating and dissolving the vinyl alcohol-based polymer in a mixed solvent of dimethyl sulfoxide and water, and cooling the solution to room temperature. Specific examples of the method for freezing and thawing the aqueous solution of the vinyl alcohol-based polymer include a method in which the aqueous solution of the vinyl alcohol-based polymer is frozen at -20°C for 15 hours or more, then thawed at 4°C for 8 hours or more, and this freeze-thaw cycle is repeated three or more times.

Examples of the method for crosslinking the vinyl alcohol-based polymer by a covalent bond include a method of using a polyfunctional crosslinking agent that reacts with a side chain hydroxyl group of the vinyl alcohol-based polymer, and a method of introducing a functional group into the vinyl alcohol-based polymer by copolymerization or post-modification and reacting the functional group.

Methods using a polyfunctional crosslinking agent that reacts with the side chain hydroxyl group of the vinyl alcohol-based polymer include: a method in which polyfunctional aldehyde compounds such as glyoxal, malondialdehyde, or glutaraldehyde are reacted with a vinyl alcohol-based polymer under acidic conditions (polyacetal crosslinking); a method in which polyfunctional epoxy compounds such as epichlorohydrin or ethylene glycol diglycidyl ether are reacted with a vinyl alcohol-based polymer under alkaline conditions (polyether crosslinking); or a method in which polyfunctional carboxylic acid compounds such as maleic acid or succinic acid are reacted with a vinyl alcohol-based polymer (polyester crosslinking). From the viewpoint of obtaining a preferable degree of swelling and/or mechanical strength of the hydrogel, the bonding amount of the polyfunctional crosslinking agent that has undergone a crosslinking reaction to the vinyl alcohol-based polymer is preferably 0.01 to 20 mol%, more preferably 0.1 to 10 mol%, and still more preferably 0.5 to 5 mol% based on the structural unit derived from vinyl alcohol.

Examples of the methods for introducing a functional group into a vinyl alcohol-based polymer by copolymerization and reacting the functional group include a method in which a vinyl ester-based monomer and a monomer other than the vinyl ester-based monomer, which has a reactive substituent other than a hydroxyl group, are copolymerized in the preparation process of the vinyl alcohol-based polymer, the resulting copolymer is then saponified to obtain a copolymer-modified polyvinyl alcohol (hereinafter may be abbreviated as "copolymer-modified PVA"), and then the copolymer-modified PVA is crosslinked with a polyfunctional crosslinking agent that reacts with a functional group such as a carboxy group or an amino group present in the copolymer-modified PVA. From the viewpoint of obtaining a preferable degree of swelling and/or mechanical strength of the hydrogel, the amount of the monomer having a reactive substituent other than the hydroxyl group based on the total structural units of the vinyl alcohol-based polymer is preferably 0.01 to 20 mol%, more preferably 0.1 to 10 mol%, and still more preferably 0.5 to 5 mol%.

Hereinafter, the copolymer-modified PVA having a carboxy group may be referred to as "carboxylic acid-modified PVA", and the copolymer-modified PVA having an amino group may be referred to as "amino-modified PVA".

Examples of monomers other than vinyl ester-based monomers that constitute carboxylic acid-modified PVA include: $\alpha$, $\beta$-unsaturated carboxylic acids such as (meth)acrylic acid, maleic acid, fumaric acid, and itaconic acid; (meth) acrylic acid alkyl esters such as methyl (meth)acrylate and ethyl (meth)acrylate; $\alpha$, $\beta$-unsaturated carboxylic acid anhydrides such as maleic anhydride and itaconic anhydride; and derivatives thereof. In the present specification, (meth)acrylic acid means acrylic acid or methacrylic acid. The crosslinked body of carboxylic acid-modified PVA may be prepared by copolymerizing a vinyl ester-based monomer with a monomer other than the vinyl ester-based monomer described above, then saponifying the copolymer to prepare carboxylic acid-modified PVA, crosslinking the carboxylic acid-modified PVA with a polyfunctional crosslinking agent that reacts with the introduced carboxy groups, for example, polyfunctional epoxy compounds such as epichlorohydrin or ethylene glycol diglycidyl ether, or crosslinking the carboxylic acid-modified PVA using a combination of polyfunctional amino compounds such as ethylenediamine, polyethyleneimine or polyallylamine, and a carbodiimide condensing agent such as 1-(3-dimethylaminopropyl)-3-ethylcarbodiimide hydrochloride.

From the viewpoint of obtaining a preferable degree of swelling and/or mechanical strength of the hydrogel, the amount of the polyfunctional crosslinking agent with respect to the carboxylic acid-modified PVA is preferably 0.01 to 20 mol%, more preferably 0.1 to 10 mol%, and still more preferably 0.5 to 5 mol% based on the total structural units of the vinyl alcohol-based polymer. The amount of the carbodiimide condensing agent based on the carboxy group of the carboxylic acid-modified PVA is preferably 0.1 to 300 mol%, more preferably 1 to 200 mol%, and still more preferably 10 to 100 mol%.

In the case of preparing a hydrogel in which a cell adhesion factor is conjugated to a crosslinked body of a vinyl alcohol-based polymer as described later, the hydrogel may be prepared by covalently bonding a carboxy group introduced into the carboxylic acid-modified PVA with the amino group of the cell adhesion factor to form an amide bond (-CONH-).

A crosslinked body of amino-modified PVA may be prepared by copolymerizing a vinyl ester-based monomer with N-vinylformamide, then saponifying the resultant to prepare amino-modified PVA, and crosslinking the amino-modified PVA by a combination of a polyfunctional crosslinking agent (for example, a polyfunctional carboxylic acid compound such as the polyfunctional epoxy compound, succinic acid, or maleic acid) that reacts with an introduced amino group and the carbodiimide condensing agent. From the viewpoint of obtaining a preferable degree of swelling and/or mechanical strength of the hydrogel, the amount of the polyfunctional crosslinking agent with respect to the amino-modified PVA is preferably 0.01 to 20 mol%, more preferably 0.1 to 10 mol%, and still more preferably 0.2 to 5 mol% based on the total structural units of the vinyl alcohol-based polymer. The amount of the carbodiimide condensing agent based on the amino groups of the amino-modified PVA is preferably 0.1 to 300 mol%, more preferably 1 to 200 mol%, and still more preferably 10 to 100 mol%.

Examples of a method for introducing a functional group into a vinyl alcohol-based polymer by post-modification and reacting the functional group include a method for introducing an ethylenically unsaturated group into a side chain of a vinyl alcohol-based polymer. The introduced ethylenically unsaturated group easily reacts by inducing a polymerization reaction by adding an additive such as a radical initiator, whereby a crosslinked body of the vinyl alcohol-based polymer can be prepared. When the vinyl alcohol-based polymer has an ethylenically unsaturated group, various molding methods including a method using a 3D printer become possible. Therefore, in one preferable embodiment, the vinyl alcohol-based polymer has an ethylenically unsaturated group. The ethylenically unsaturated group is preferably a radical-polymerizable group, and specific examples thereof include vinyl groups, (meth)acryloyl groups, and (meth)acryloylamino groups; cyclic unsaturated hydrocarbon groups such as vinylphenyl group, cyclohexenyl group, cyclopentenyl group, norbornenyl group, and dicyclopentenyl group; and derivatives thereof. The presence of ethylenically unsaturated groups in the vinyl alcohol-based polymer may be confirmed by, for example, nuclear magnetic resonance (NMR)analysis.

The ethylenically unsaturated group is preferably introduced via a side chain or terminal functional group of the vinyl alcohol-based polymer, and more preferably introduced by reacting the hydroxyl groups on the side chains of the vinyl alcohol-based polymer with a compound containing an ethylenically unsaturated group (hereinafter, may be abbreviated as "ethylenically unsaturated group-containing compound").

Examples of ethylenically unsaturated group-containing compounds that react with the hydroxyl groups on the side chains of the vinyl alcohol-based polymer include (meth)acrylic acid or derivatives thereof, such as (meth)acrylic acid, (meth)acrylic anhydride, (meth)acrylic acid halides, and (meth)acrylic acid esters. By subjecting these compounds preferably to an esterification reaction or transesterification reaction in the presence of a base, (meth)acryloyl groups can be introduced into the vinyl alcohol-based polymer.

Examples of ethylenically unsaturated group-containing compounds that react with the hydroxyl groups on the side chains of the vinyl alcohol-based polymer also include compounds having both an ethylenically unsaturated group and a glycidyl group in the molecule, and specific examples thereof include glycidyl (meth)acrylate and allyl glycidyl ether. By subjecting these compounds to an etherification reaction in the presence of a base, (meth)acryloyl groups or allyl groups can be introduced into the vinyl alcohol-based polymer.

Examples of ethylenically unsaturated group-containing compounds that react with the 1,3-diol groups of the vinyl alcohol-based polymer include compounds having both an ethylenically unsaturated group and an aldehyde group in the molecule, such as acrylic aldehyde (acrolein), methacrylic aldehyde (methacrolein), 5-norbornene-2-carboxaldehyde, 7-octenal, 3-vinylbenzaldehyde, and 4-vinylbenzaldehyde. By subjecting these compounds to an acetalization reaction in the presence of an acid catalyst, an ethylenically unsaturated group can be introduced into the vinyl alcohol-based polymer. More specifically, for example, by subjecting 5-norbornene-2-carboxaldehyde, 3-vinylbenzaldehyde, or 4-vinylbenzaldehyde to an acetalization reaction, norbornenyl groups or vinylphenyl groups can be introduced into the vinyl alcohol-based polymer. Further, by reacting N-(2,2-dimethoxyethyl) (meth)acrylamide, (meth)acryloylamino groups can be introduced into the vinyl alcohol-based polymer.

Methods for introducing ethylenically unsaturated groups into a vinyl alcohol-based polymer are not limited to the aforementioned reactions, and two or more such reactions may be used in combination.

Examples of another method for introducing the ethylenically unsaturated group include a method in which a reactive substituent such as a carboxy group present in carboxylic acid-modified PVA or an amino group present in amino-modified PVA is reacted with an ethylenically unsaturated group-containing compound. For the carboxy groups of carboxylic acid-modified PVA, for example, by reacting glycidyl methacrylate under acidic conditions to form an ester bond, methacryloyl groups can be introduced into the carboxylic acid-modified PVA. For the amino groups of amino-modified PVA, for example, acryloylamino groups can be introduced by subjecting acrylic anhydride to an amidation reaction in the presence of a base, and vinyl oxycarbonyl groups can be introduced by subjecting divinyl adipate to an amidation reaction. Method for introducing ethylenically unsaturated groups via copolymer-modified PVA are not limited to the aforementioned reactions, and two or more such reactions may be used in combination.

From the viewpoint of ease of preparation, preferred vinyl alcohol-based polymers having ethylenically unsaturated groups are those in which the ethylenically unsaturated groups are introduced via side-chain hydroxyl groups such as hydroxyl groups or 1,3-diol groups in the side chains. More preferred are vinyl alcohol-based polymer in which (meth)acrylic acid or a derivative thereof is subjected to an esterification or transesterification reaction with the side-chain hydroxyl group, or one in which a compound having both an ethylenically unsaturated group and an aldehyde group in the molecule is subjected to an acetalization reaction with the 1,3-diol group.

From the viewpoint of suppressing embrittlement of the hydrogel, the introduction rate of the ethylenically unsaturated group is preferably 10 mol% or less, more preferably 5 mol% or less, and still more preferably 3 mol% or less based on the total structural units constituting the vinyl alcohol-based polymer. From the viewpoint of promoting the crosslinking reaction and rapidly forming the hydrogel, as well as improving the elastic modulus of the obtained hydrogel, the introduction rate is preferably 0.01 mol% or more, more preferably 0.1 mol% or more, and still more preferably 0.5 mol% or more.

From the viewpoint of improving the mechanical strength of the hydrogel and introducing a functional group different from a hydroxyl group, the vinyl alcohol-based polymer having an ethylenically unsaturated group may further contain a structural unit derived from another monomer. Examples of the other monomers include: acrylamides such as acrylamide, N-isopropylacrylamide, 2-acrylamido-2-methylpropanesulfonic acid, and N,N-dimethylacrylamide; $\alpha,\beta$-unsaturated carboxylic acids such as (meth)acrylic acid, crotonic acid, itaconic acid, maleic acid, and fumaric acid; water-soluble radical-polymerizable monomers such as vinylpyridine, hydroxyethyl (meth)acrylate, styrenesulfonic acid, and polyethylene glycol mono(meth)acrylate; and compounds having two or more ethylenically unsaturated groups in the molecule, such as N,N'-methylenebisacrylamide, ethylene glycol di(meth)acrylate, diethylene glycol di(meth)acrylate, and polyethylene glycol di(meth)acrylate. From the viewpoint of improving the mechanical strength of the hydrogel, the content of another monomer is preferably 50 mass% or less, more preferably 30 mass% or less, and still more preferably 10 mass% or less based on the total mass of the vinyl alcohol-based polymer having an ethylenically unsaturated group.

When active energy rays or heat are applied to a vinyl alcohol-based polymer having an ethylenically unsaturated group, the ethylenically unsaturated group in the vinyl alcohol-based polymer is crosslinked, whereby a crosslinked body of the vinyl alcohol-based polymer can be obtained. Examples of the active energy rays include gamma rays, ultraviolet rays, visible light, infrared rays (heat rays), radio waves, alpha rays, beta rays, electron beams, plasma streams, ionizing radiation, and particle beams. When the vinyl alcohol-based polymer is crosslinked by ultraviolet rays, visible light, infrared rays (heat rays) among the active energy rays, or heat, the above-mentioned uncrosslinked polymer solution preferably contains a radical polymerization initiator. Examples of the radical polymerization initiator include a photoradical polymerization initiator and a thermal radical polymerization initiator.

The photoradical polymerization initiator is not particularly limited as long as radical polymerization is initiated by irradiation with active energy rays such as ultraviolet rays and visible light. From the viewpoint of enabling the photoradical polymerization initiator to be washed and removed after crosslinking, it is preferable that the photoradical polymerization initiator is water-soluble. Specific examples of photoradical polymerization initiators include 1-[4-(2-hydroxyethoxy)-phenyl]-2-hydroxy-2-methyl-1-propan-1-one (trade name "Omnirad2959", manufactured by IGM RESINS B.V.), $\alpha$-ketoglutaric acid, phenyl (2,4,6-trimethylbenzoyl) phosphine lithium salt (trade name "L0290", manufactured by Tokyo Chemical Industry Co., Ltd.), and Eosin Y. When the photoradical polymerization initiator is used, the amount thereof is usually 0.0001 to 10 parts by mass, preferably 0.001 to 5 parts by mass, and more preferably 0.005 to 3 parts by mass, based on 100 parts by mass of the vinyl alcohol-based polymer.

The thermal radical polymerization initiator is not particularly limited as long as radical polymerization is initiated by heat. Examples thereof include azo-based initiators and peroxide-based initiators which are generally used in radical polymerization. When the thermal radical polymerization initiator is used, the amount thereof is usually 0.001 to 30 parts by mass, preferably 0.01 to 10 parts by mass, and more preferably 0.1 to 5 parts by mass, based on 100 parts by mass of the vinyl alcohol-based polymer.

It is preferable that the azo-based initiator is water-soluble from the viewpoint of being able to wash and remove the azo-based initiator after crosslinking. Specific examples thereof include 2,2'-azobis[2-(2-imidazolin-2-yl)propane] dihydrochloride (trade name "VA-044"), 2,2'-azobis[2-(2-imidazolin-2-yl)propane] disulfate dihydrate (trade name "VA-044B"), 2,2'-azobis[2-methylpropionamidine] dihydrochloride (trade name "V-50"), 2,2'-azobis[N-(2-carboxyethyl)-2-methylpropionamidine] tetrahydrate (trade name "VA-057"), 2,2'-azobis[2-(2-imidazolin-2-yl)propane] (trade name "VA-061"), 2,2'-azobis[2-methyl-N-(2-hydroxyethyl)propionamide] (trade name "VA-086"), and 4,4'-azobis(4-cyanopentanoic acid) (trade name "V-501") (all manufactured by Wako Pure Chemical Corporation).

As the peroxide-based initiator, a peroxide-based initiator that does not generate gas is preferable from the viewpoint of improving the physical properties of the vinyl alcohol-based polymer, and a peroxide-based initiator that is water-soluble is preferable from the viewpoint of being able to wash and remove the thermal radical polymerization initiator after crosslinking. Specific examples thereof include inorganic peroxides such as ammonium persulfate, potassium persulfate, and sodium persulfate.

In addition, a redox-based polymerization initiator that is a combination of a peroxide-based initiator and a reducing agent may be used. As the reducing agent, a known reducing agent may be used. Preferable examples of reducing agents include highly water-soluble compounds such as N,N,N',N'-tetramethylethylenediamine, sodium sulfite, sodium bisulfite, and sodium hydrosulfite. When the redox-based polymerization initiator is used, the amount thereof is preferably 0.001 to 30 parts by mass, more preferably 0.01 to 10 parts by mass, and still more preferably 0.1 to 5 parts by mass, based on 100 parts by mass of the vinyl alcohol-based polymer.

When a vinyl alcohol-based polymer having a vinyl group as an ethylenically unsaturated group is crosslinked, from the viewpoint of promoting crosslinking, for example, a polythiol having two or more thiol groups in the molecule may be added, and crosslinking may be performed using a thiol-ene reaction. As the polythiol, those being water-soluble are preferable, and examples thereof include polythiols having hydroxyl groups such as dithiothreitol; polythiols containing ether linkages such as 3,6-dioxa-1,8-octanedithiol, polyethylene glycol dithiol, and thiol-terminated multi-arm polyethylene glycol. When a polythiol is used, the amount thereof is preferably 0.01 to 20 mol%, more preferably 0.1 to 10 mol%, and still more preferably 0.5 to 5 mol% based on the total structural units of the vinyl alcohol-based polymer.

The method for crosslinking the vinyl alcohol-based polymer by a covalent bond may be appropriately selected from the methods exemplified above. From the viewpoint of the toxicity of the unreacted crosslinking agent or the condensing agent contained in the hydrogel and the ease of molding, a method for introducing a functional group into the vinyl alcohol-based polymer by post-modification to react the functional group, particularly a method for introducing an ethylenically unsaturated group into the side chain of the vinyl alcohol-based polymer by post-modification is preferable.

Before the molding step, the uncrosslinked polymer solution may contain a component necessary for crosslinking, such as the crosslinking agent or the initiator, in addition to the vinyl alcohol-based polymer. When the molded vinyl alcohol-based polymer is dried as described above, a component necessary for crosslinking may be added after molding. Although the crosslinking conditions may be optimally selected depending on the vinyl alcohol-based polymer or the crosslinking method as described above, it is preferable to perform molding before completion of crosslinking, since molding is difficult after crosslinking is completed. When a component necessary for crosslinking is mixed with the uncrosslinked polymer solution before the molding step, it is preferable to mix the component immediately before the molding step. When a cell adhesion factor is contained before crosslinking of the vinyl alcohol-based polymer and the crosslinking requires heat, the temperature of the crosslinking reaction is preferably 100°C or lower, more preferably 60°C or lower, and still more preferably 37°C or lower from the viewpoint of maintaining the activity of the cell adhesion factor.

After the crosslinking step, the crosslinked body of the vinyl alcohol-based polymer may be washed with a washing liquid as necessary. When crosslinking is performed using a water-soluble photoradical polymerization initiator or a thermal radical polymerization initiator, the photoradical polymerization initiator or the thermal radical polymerization initiator can be removed by washing. Washing may be performed by a general method and may be performed one or more times. Examples of the washing liquid include one or more selected from the group consisting of water and the water-soluble organic solvent, and water is preferable. When the washing liquid is water, a buffer solution may also be used in combination, for example, phosphate buffer, Tris buffer, HEPES buffer, acetate buffer, citrate buffer, bicarbonate buffer, or MES buffer.

(Cell Adhesion Factor)

In one preferred embodiment, the hydrogel contains a cell adhesion factor. In this case, the hydrogel may be a hydrogel in which the crosslinked body of the vinyl alcohol-based polymer simply contains the cell adhesion factor, or may be a complex in which the cell adhesion factor is covalently bonded to the crosslinked body of the vinyl alcohol-based polymer. From the viewpoint of allowing the cell adhesion factor to function stably and of enabling the hydrogel to retain he cell adhesion factor more effectively after the separation step described later, it is preferable that the cell adhesion factor is in the form of a complex.

The cell adhesion factor is not particularly limited as long as it is a molecule having cell adhesion properties. Examples thereof include cell adhesive proteins such as gelatin, collagen, laminin, fibronectin, vitronectin, nidogen, tenascin, thrombospondin, von Willebrand factor, osteopontin, fibrinogen, fibrin, elastin, netrin, entactin, proteoglycan, and retronectin; synthetic peptides containing cell-adhesive sequences such as RGD, RGDVF, REDV, YIGSR, IKVAV, and peptides; acidic polysaccharides containing glycosaminoglycans such as heparin, hyaluronic acid, chondroitin sulfate, dermatan sulfate, and heparan sulfate; polycations such as $\alpha$-polylysine, $\varepsilon$-polylysine, $\alpha$-polyarginine, and chitosan; antibodies such as anti-integrin antibody, anti-VCAM1 antibody, anti-E-selectin antibody, anti-CD3 antibody, and anti-CD28 antibody; cell growth factors/differentiation factors described later; and cytokines described later.

The cell adhesive protein may be a naturally occurring protein, a recombinant type, or a synthetic peptide chemically synthesized by a method such as solid phase method. When a cell adhesive protein is used as the cell adhesion factor, the molecular weight of thereof is not particularly limited, but is preferably from 5,000 to 1,000,000, and more preferably from 6,000 to 800,000.

When a synthetic peptide is used as the cell adhesion factor, the molecular weight thereof is not particularly limited, but is preferably from 200 to 10,000, and more preferably from 250 to 7,000.

When an acidic polysaccharide is used as the cell adhesion factor, the molecular weight thereof is not particularly limited, but is preferably from 3,000 to 2,000,000, and more preferably from 4,000 to 1,200,000.

When a polycation is used as the cell adhesion factor, the molecular weight thereof is not particularly limited, but is preferably from 2,000 to 1,000,000, and more preferably from 4,000 to 500,000.

When the cell growth factors/differentiation factors are used as cell adhesion factors, the molecular weight thereof is not particularly limited, but is preferably from 3,000 to 200,000, and more preferably from 5,000 to 100,000.

When the hydrogel contains a cell adhesion factor, the cell adhesion factor may be contained in the hydrogel alone or in combination of two or more.

When the hydrogel contains the cell adhesion factor and the crosslinked body of the vinyl alcohol-based polymer simply contains the cell adhesion factor, the hydrogel containing the cell adhesion factor can be prepared by mixing the crosslinked body of the vinyl alcohol-based polymer and the cell adhesion factor, or by mixing the vinyl alcohol-based polymer and the cell adhesion factor and then physically crosslinking or crosslinking by a covalent bond as described above. From the viewpoint of eliminating the possibility of activity reduction or loss of the cell adhesion factor due to crosslinking, it is preferable to prepare the hydrogel containing the cell adhesion factor by mixing the crosslinked body of the vinyl alcohol-based polymer with the cell adhesion factor.

A hydrogel that contains a cell adhesion factor and in which the cell adhesion factor is covalently bonded to the crosslinked body of the vinyl alcohol-based polymer (that is, the cell adhesion factor is conjugated to the crosslinked body of the vinyl alcohol-based polymer) can be prepared by: forming a covalent bond by activating the hydroxyl groups of the vinyl alcohol-based polymer and reacting them with the functional groups of the cell adhesion factor, followed by the above-described crosslinking; forming a covalent bond by reacting the functional groups of carboxylic acid-modified PVA or amino-modified PVA with the functional groups of the cell adhesion factor, followed by the above-described crosslinking; forming a covalent bond by reacting the residual functional groups in the crosslinked body of carboxylic acid-modified PVA or amino-modified PVA with the functional groups of the cell adhesion factor; or first introducing functional groups for conjugation to hydroxyl groups contained in the crosslinked body of the vinyl alcohol-based polymer, and then forming a covalent bond by reacting the introduced functional groups with the functional groups of the cell adhesion factor. From the viewpoint of eliminating the possibility of activity reduction or loss of the cell adhesion factor due to crosslinking, it is preferable to prepare a hydrogel containing the cell adhesion factor by reacting the functional groups of the crosslinked body with the functional groups of the cell adhesion factor. From the viewpoint of reaction efficiency, the functional group of the cell adhesion factor is preferably an amino group, a carboxy group, or a thiol group.

As a method for introducing a cell adhesion factor by activating the hydroxyl groups of the vinyl alcohol-based polymer and reacting them with functional groups of the cell adhesion factor, examples thereof include methods using hydroxyl group-activating reagents such as 1,1'-carbonyldiimidazole, di(N-succinimidyl) carbonate, p-toluenesulfonyl chloride, 2,2,2-trifluoroethanesulfonyl chloride, and cyanuric chloride. When these hydroxyl group activating reagents are reacted with the hydroxyl group of the vinyl alcohol-based polymer, a covalent bond between the activated hydroxyl group and a functional group such as a primary or secondary amino group, a carboxy group, or a hydroxyl group of the cell adhesion factor may be formed. In addition, it is also possible to use an acid anhydride reagent such as succinic anhydride capable of introducing a carboxy group into a hydroxyl group of a vinyl alcohol-based polymer via an ester bond, and an acetal reagent such as 2,2-dimethoxyethylamine capable of introducing an amino group into a 1,3-diol group of a vinyl alcohol-based polymer via an acetal bond. The introduced carboxy group and amino group can form an amide bond with the amino group or carboxy group of the cell adhesion factor by using the carbodiimide condensing agent. The introduction rate of the carboxy group introduced into the hydroxyl group of the vinyl alcohol-based polymer via an ester bond is preferably 50 mol% or less, more preferably 30 mol% or less, and still more preferably 15 mol% or less in all structural units constituting the vinyl alcohol-based polymer from the viewpoint of appropriately maintaining the swelling properties of the hydrogel in a solvent and maintaining physical properties such as gel strength. Then, the immobilization amount of the cell adhesion factor is preferably 0.1 mol% or more, more preferably 0.5 mol% or more, and still more preferably 1.0 mol% or more from the viewpoint of being able to set the immobilization amount to an appropriate range and expressing a high function. The amount of the hydroxyl group-activating reagent, acid anhydride reagent, or acetal reagent is typically 0.01 to 30 mol%, and preferably 0.1 to 15 mol%, based on the total structural units of the vinyl alcohol-based polymer.

Examples of the method for introducing the cell adhesion factor into the carboxylic acid-modified PVA or the amino-modified PVA include a method in which the carboxy group of the carboxylic acid-modified PVA or the amino group of the amino-modified PVA is amide-bonded to the amino group or the carboxy group of the cell adhesion factor by using the carbodiimide condensing agent. The amount of the carbodiimide condensing agent is usually 0.1 to 300 mol%, and preferably 1 to 200 mol%, based on the carboxy group or the amino group of the carboxylic acid-modified PVA or the amino-modified PVA.

Examples of the method for forming a covalent bond by reacting the functional group remaining in the carboxylic acid-modified PVA crosslinked body or the amino-modified PVA crosslinked body with the functional group of the cell adhesion factor include a method using the carbodiimide condensing agent. The amount of the carbodiimide condensing agent is usually 0.1 to 300 mol%, and preferably 1 to 200 mol%, based on the carboxy group or the amino group of the carboxylic acid-modified PVA or the amino-modified PVA.

Examples of the method for forming a covalent bond by first introducing a functional group for conjugation to hydroxyl groups contained in the crosslinked body of the vinyl alcohol-based polymer, and then reacting the introduced functional group with the functional group of a cell adhesion factor include a method in which a functional group is first introduced using a hydroxyl group-activating reagent, acid anhydride reagent, or acetal reagent as described above, and then the introduced functional group is reacted with the functional group of the cell adhesion factor using a carbodiimide condensing agent or the like. The amount of the hydroxyl group-activating reagent, acid anhydride reagent, or acetal reagent is typically 0.01 to 30 mol%, and preferably 0.1 to 15 mol%, based on all structural units of the crosslinked body of the vinyl alcohol-based polymer. The amount of the carbodiimide condensing agent is usually 0.1 to 300 mol%, and preferably 1 to 200 mol%, based on the carboxy group or the amino group of the carboxylic acid-modified PVA or the amino-modified PVA. For example, when an acid anhydride reagent such as succinic anhydride is reacted with the crosslinked body of a vinyl alcohol-based polymer, a carboxy group (COOH) derived from succinic acid is introduced at the terminal, and this carboxy group can be condensed with an amino group of a cell adhesion factor using a condensing agent such as 1,1'-carbonyldiimidazole, dicyclohexylcarbodiimide, or a water-soluble carbodiimide (for example, 1-(3-dimethylaminopropyl)-3-ethylcarbodiimide hydrochloride), thereby allowing the cell adhesion factor to be conjugated to the crosslinked body of the vinyl alcohol-based polymer.

The carboxylic acid-modified PVA may be subjected to active esterification with an alcohol and a carbodiimide condensing agent, and then the active ester may be condensed with the amino group of the cell adhesion factor. Examples of alcohols used for active esterification include N-hydroxysuccinimide, sodium N-hydroxysulfosuccinimide, 1-hydroxybenzotriazole, 1-hydroxy-7-azabenzotriazole, and ethyl (hydroxyimino)cyanoacetate. Considering factors such as water solubility, N-hydroxysuccinimide and sodium N-hydroxysulfosuccinimide are preferable. The amount of alcohol used for the active esterification is usually 0.1 to 300 mol%, and preferably 1 to 200 mol%, based on the carboxy group of the carboxylic acid-modified PVA.

The proportion of the crosslinked body of the vinyl alcohol-based polymer in the hydrogel is, for example, 50 to 100 mass%, preferably 70 to 98 mass%, and more preferably 85 to 97 mass% based on the total mass of the hydrogel. In this context, the mass of the hydrogel and the crosslinked body of the vinyl alcohol-based polymer refers to the mass in a dry state.

When the hydrogel contains a cell adhesion factor, the proportion of the cell adhesion factor in the hydrogel is, for example, 0.00001 to 70 mass%, preferably 0.0001 to 60 mass%, and more preferably 0.0005 to 50 mass%, based on the total mass of the hydrogel. In this context, the mass of the hydrogel and the cell adhesion factor refers to the mass in a dry state.

For the proportion of the crosslinked body of vinyl alcohol-based polymer and the proportion of the cell adhesion factor in the hydrogel when the hydrogel contains the cell adhesion factor, for example, the proportion of the cell adhesion factor is determined by a ninhydrin method, a Lowry method, a BCA method, an ELISA method, a radiolabeling method, and the amount of the cell adhesion factor is subtracted from the total amount of the hydrogel, whereby the proportion of the crosslinked body of the vinyl alcohol-based polymer can be determined.

A preferred hydrogel is one in which a crosslinked body of a vinyl alcohol-based polymer having ethylenically unsaturated groups and carboxy groups and a cell adhesion factor having amino groups are covalently bonded. More specifically, a hydrogel in which a carboxy group of a carboxy group-containing vinyl alcohol-based polymer crosslinked by an ethylenically unsaturated group and an amino group of a cell adhesion factor are amide-bonded is more preferable. By forming an amide bond, the cell adhesion factor is retained in the hydrogel and can function stably.

(Optional Component Other Than Cell Adhesion Factor)

Examples of the optional component other than the cell adhesion factor which may be contained, as needed, in the hydrogel include polymer fine particles and inorganic fine particles. These optional components may be included alone or in combination of two or more thereof.

When the hydrogel contains the optional component, a hydrogel containing the optional component can be prepared by mixing the optional component with components other than the optional component. The mixing method is not particularly limited, and may be appropriately selected. For example, the optional component may be mixed during the mixing of the crosslinked body of the vinyl alcohol-based polymer and the cell adhesion factor; the optional component may be mixed with a complex in which the cell adhesion factor is covalently bonded to the crosslinked body of the vinyl alcohol-based polymer; or the optional component may be added during the preparation of the uncrosslinked polymer solution.

The proportion of the optional component in the hydrogel can be appropriately selected. When the hydrogel contains one or more selected from the group consisting of polymer fine particles and inorganic fine particles, the proportion thereof is preferably 0.1 to 20 parts by mass, more preferably 0.5 to 20 parts by mass, and still more preferably 1 to 15 parts by mass based on 100 parts by mass of the vinyl alcohol-based polymer from the viewpoint of being able to improve the mechanical strength of the hydrogel. The mass of the hydrogel and the optional component at this time is the mass in a dry state.

(Drying Step)

The hydrogel obtained after the crosslinking step contains the solvent used in the uncrosslinked polymer solution preparation step. As described above, the hydrogel before being mixed or introduced in the preparation step in the present invention may be in a dry state, may contain a liquid such as a solvent (water, water-soluble organic solvent) or a liquid medium, or may be in an equilibrium swollen state by the liquid.

In one preferable embodiment, the prepared hydrogel is subjected to a drying treatment to remove the solvent. The drying method is not particularly limited, and general methods such as hot air drying, air drying, vacuum drying, reduced pressure drying, and freeze drying may be adopted. In addition, it is also possible to immerse the hydrogel in a volatile water-soluble organic solvent to remove components such as water contained in the hydrogel, and then dry the hydrogel. Examples of volatile water-soluble organic solvents include aprotic polar solvents such as dimethylformamide, dimethylacetamide, dimethyl sulfoxide, and N-methylpyrrolidone; monoalcohols such as methanol, ethanol, propanol, and isopropanol; and acetone. The drying conditions may be appropriately selected. The water content of the hydrogel after the drying treatment represented by the following Equation (3) is preferably 75 mass% or less, more preferably 60 mass% or less, and still more preferably 40 mass% or less.

Water content [%] = [(Mass of water contained in hydrogel)/(Mass of hydrogel in dry state + Mass of water contained in hydrogel)] x 100      Equation (3)

An appropriate method for measuring the water content of the hydrogel varies depending on the type of solvent contained in the hydrogel. When the hydrogel contains only water as a solvent, the water content of the hydrogel can be calculated using the heating and drying type moisture analyzer to directly evaporate the water contained in the hydrogel and measuring the mass of the remaining hydrogel in a dry state. When the hydrogel contains water and the water-soluble organic solvent, the water content can be measured by subjecting the hydrogel to $^1$H-NMR measurement. Specifically, the hydrogel is swollen with heavy DMSO to obtain a $^1$H-NMR spectrum and an integral value, and the integral values of peaks derived from a crosslinked body of a vinyl alcohol-based polymer are compared, whereby the contents of water and the water-soluble organic solvent can be calculated. First, the presence of the water-soluble organic solvent is confirmed by $^1$H-NMR, and when only water is found to be present as a solvent, then measuring the water content using a heating and drying type moisture analyzer is one preferable method for determining the water content.

(Sterilization Step)

The hydrogel is preferably subjected to a sterilization treatment such that the sterility assurance level described above is achieved. The sterilization treatment is usually performed after the drying treatment described above. When the hydrogel contains a cell adhesion factor, sterilizing the hydrogel in a water-containing state may reduce or eliminate the activity of the cell adhesion factor, and thus, it is preferable to perform sterilization after drying treatment.

The sterilization method is not particularly limited. Specific examples of sterilization methods include autoclave sterilization, ethylene oxide gas sterilization, hydrogen peroxide low-temperature plasma sterilization, dry heat sterilization, chemical sterilization using glutaraldehyde or the like, and radiation sterilization using gamma rays or electron beams. Among these, when the hydrogel contains a cell adhesion factor, from the viewpoint of maintaining the activity of the factor, ethylene oxide gas sterilization, hydrogen peroxide low-temperature plasma sterilization, and radiation sterilization are preferable, and radiation sterilization is more preferable from the viewpoint of avoiding residues.

<Liquid Medium>

The liquid medium to be used is not particularly limited, and one suitable for cells can be freely selected and used. Examples of liquid media include Dulbecco's Modified Eagles's Medium (DMEM), Ham's Nutrient Mixture F12, DMEM/F12 medium, McCoy's 5A medium, Eagles's Minimum Essential Medium (EMEM), alpha Modified Eagles's Minimum Essential Medium ($\alpha$MEM), Minimum Essential Medium (MEM), RPM11640 medium, and Iscove's Modified Dulbecco's Medium (IMDM), and one of these or a mixture of two or more may be used as the liquid medium. Of course, in addition to this, a medium suitable for cells to be cultured can be used. Fetal bovine serum may be added to the liquid medium, or the medium may be xenogeneic component-free (xeno-free) without the addition of fetal bovine serum, and a fully synthetic medium that contains neither fetal bovine serum nor protein components or animal-derived components may also be used.

<Raw Material Cells>

The raw material cells to be used are those employed as raw materials in the production method of the present invention and are capable of proliferating on the surface of the hydrogel used in the present invention. Examples thereof include pluripotent stem cells, tissue stem cells, somatic cells, mammalian-derived established cell lines used for the production of useful substances such as pharmaceutical products or for therapeutic purposes, and insect cells.

The pluripotent stem cells are stem cells having the ability to differentiate into cells of any tissue (pluripotency), and are, for example, embryonic stem cells (ES cells), induced pluripotent stem cells (iPS cells), embryonic germ stem cells (EG cells), germ stem cells (GS cells).

The tissue stem cell means a stem cell having the ability to differentiate into various cell types (pluripotency) although the tissue to be differentiated is limited. Examples thereof include bone marrow-undifferentiated mesenchymal stem cells, skeletal muscle stem cells, hematopoietic stem cells, neural stem cells, hepatic stem cells, adipose tissue stem cells, epidermal stem cells, intestinal stem cells, spermatogonial stem cells, pancreatic stem cells (such as pancreatic duct epithelial stem cells), leukocyte lineage stem cells, lymphocyte lineage stem cells, and corneal lineage stem cells.

Somatic cells refer to the cells that make up multicellular organisms. Examples thereof include osteoblasts, chondrocytes, hematopoietic cells, epithelial cells (for example, mammary gland epithelial cells), endothelial cells (for example, vascular endothelial cells), epidermal cells, fibroblasts, mesenchymal-derived cells, cardiomyocytes, myoblasts, smooth muscle cells, skeletal muscle cells derived from living organisms, human tumor cells, fibrocytes, EB virus-transformed cells, hepatocytes, renal cells, bone marrow cells, macrophages, hepatic parenchymal cells, small intestinal cells, mammary gland cells, salivary gland cells, thyroid cells, skin cells, plasma cells, T cells, B cells, killer cells, lymphoblasts, and pancreatic $\beta$-cells.

Examples of mammalian-derived established cell lines include CRFK cells, 3T3 cells, A549 cells, AH130 cells, B95-8 cells, BHK cells, BOSC23 cells, BS-C-1 cells, C3H10T1/2 cells, C-6 cells, CHO cells, COS cells, CV-1 cells, F9 cells, FL cells, FL5-1 cells, FM3A cells, G-361 cells, GP+E-86 cells, GP+envAm12 cells, H4-II-E cells, HEK293 cells, HeLa cells, HEp-2 cells, HL-60 cells, HTC cells, HUVEC cells, IMR-32 cells, IMR-90 cells, K562 cells, KB cells, L cells, L5178Y cells, L-929 cells, MA104 cells, MDBK cells, MDCK cells, MIA PaCG-2 cells, N18 cells, Namalwa cells, NG108-15 cells, NRK cells, OC10 cells, OTT6050 cells, P388 cells, PA12 cells, PA317 cells, PC-12 cells, PER.C6 cells, PG13 cells, QGH cells, Raji cells, RPMI-1788 cells, SGE1 cells, Sp2/O-Ag14 cells, ST2 cells, THP-1 cells, U-937 cells, V79 cells, VERO cells, WI-38 cells, $\psi$2 cells, and $\psi$CRE cells.

Examples of insect cells include silkworm cells (for example, BmN cells and BoMo cells), *Bombyx mandarina* cells, tussah cells, *ailanthus* moth cells, cabbage armyworm cells (e.g., Sf9 cells and Sf21 cells), *Lemyra imparilis* cells, leaf roller cells, vinegar fly cells, *Boettcherisca peregrine* cells, *Aedes albopictus* cells, *Papilio xuthus* cells, American cockroach cells, and *Trichoplusia ni* cells (e.g., Tn-5 cells, High Five cells and MG1 cells).

These cells may be aggregated with each other or may be differentiated. The aggregated cells may have a function as an organ. The cells may be those immediately after being collected from a living body or may be cultured. The cells collected from the living body may form an organ.

<Optional Component>

Examples of optional components that may be included in the composition contained in the culture vessel include serum substitutes, cell growth factors/differentiation factors, cytokines, hormones, polyanions, polyvinyl alcohol, and transferrin. These optional components can be used alone or in combination of two or more thereof.

Examples of serum substitutes include insulin, growth factors/differentiation factors, transferrin, and furthermore, proteins such as albumin.

Examples of cell growth factors/differentiation factors include vascular endothelial growth factor (VEGF), basic fibroblast growth factor (bFGF), transforming growth factor-$\beta$ (TGF-$\beta$), osteonectin, angiopoietin, hepatocyte growth factor (HGF), epidermal growth factor (EGF), platelet-derived growth factor (PDGF), insulin-like growth factor (IGF), brain-derived neurotrophic factor (BDNF), ciliary neurotrophic factor (CNTF), glial cell line-derived neurotrophic factor (GDNF), nerve growth factor (NGF), leukemia inhibitory factor (LIF), stem cell factor (SCF), bone morphogenetic protein (BMP), interferon-$\alpha$, interferon-$\beta$, interferon-$\gamma$, tumor necrosis factor-a (TNF-$\alpha$), tumor necrosis factor-$\beta$ (TNF-$\beta$), and Notch ligands such as Delta-1, Delta-3, Delta-4, Jagged-1, and Jagged-2.

Examples of cytokines include interleukin-1$\alpha$, interleukin-1$\beta$, interleukin-2, interleukin-4, interleukin-5, interleukin-6, interleukin-7, interleukin-8, interleukin-10, interleukin-11, interleukin-12, interleukin-13, interleukin-14, interleukin-15, interleukin-16, interleukin-17, interleukin-18, interferon-$\alpha$, interferon-$\beta$, interferon-$\gamma$, granulocyte colony-stimulating factor (G-CSF), granulocyte-macrophage colony-stimulating factor (GM-CSF), macrophage colony-stimulating factor (M-CSF), MCP-1, erythropoietin (EPO), thrombopoietin (TPO), and Flk-2/Flt-3 ligand (FL).

Examples of hormones include melatonin, serotonin, thyroxine, triiodothyronine, epinephrine, norepinephrine, dopamine, anti-Muellerian hormone, adiponectin, adrenocorticotropic hormone, angiotensinogen, angiotensin, antidiuretic hormone, atrial natriuretic peptide, calcitonin, cholecystokinin, corticotropin-releasing hormone, follicle-stimulating hormone, gastrin, ghrelin, glucagon, gonadotropinreleasing hormone, growth hormone-releasing hormone, human chorionic gonadotropin, human placental lactogen, growth hormone, inhibin, insulin, leptin, luteinizing hormone, melanocyte-stimulating hormone, oxytocin, parathyroid hormone, prolactin, secretin, somatostatin, thyroid-stimulating hormone, thyrotropin-releasing hormone, cortisol, aldosterone, testosterone, dehydroepiandrosterone, dihydrotestosterone, estradiol, estrone, estriol, progesterone, calcitriol, calcidiol, prostaglandin, leukotriene, prostacyclin, thromboxane, prolactin-releasing hormone, lipotropin, brain natriuretic peptide, neuropeptide Y, histamine, endothelin, pancreatic polypeptide, renin, and enkephalin.

Examples of polyanions include heparin, dextran sulfate, heparan sulfate, dermatan sulfate, and chondroitin sulfate. These polyanions stabilize, for example, by binding to the above-mentioned cell growth factors/differentiation factors, thereby promoting cell proliferation more efficiently.

The polyvinyl alcohol (PVA) may be freely selected from the vinyl alcohol-based polymer or modified PVA. When cells are cultured, bovine serum albumin (BSA), and/or human recombinant albumin are often added to the medium for the purpose of protecting cells, stabilizing growth factors. By using PVA, a serum-free culture environment excluding proteins such as BSA may be constructed. The content of PVA in the serum-free medium is preferably 0.01 to 0.5 mass%, and more preferably 0.05 to 0.2 mass%.

<Culture Vessel>

The culture vessel used in the present invention is not particularly limited, and a commonly used culture vessel may be used. Examples thereof include plates, well plates, flasks (including T-flasks, shaker flasks, or spinner flasks), chamber slides, dishes, tubes, bottles, roller bottles, bags (including shaking bags), and bioreactors. Both homogeneous type and heterogeneous type bioreactors may be used. In the homogeneous type bioreactor, the cells may freely contact the liquid medium and the cells are dispersed and maintained inside the reactor. As the homogeneous type bioreactor, a stirred tank type, a bubble column type, an air lift type, a shaking type may be used. In the heterogeneous type bioreactor, the cells are not in direct contact with the liquid medium, and the cells are stably adhered or retained to the cell culture carrier (the hydrogel in the present invention) and stably cultured. Examples of heterogeneous type bioreactors include fixed bed type, fluidized bed type, stirred tank type, and hollow fiber type. Here, the fixed bed type bioreactor refers to a bioreactor in which the cell culture carriers are fixed and the liquid medium flows between the cell culture carriers. The fluidized bed type bioreactor is a bioreactor in which a cell culture carrier is not immobilized and flows inside the reactor by upward flow of a liquid medium. The stirred tank type bioreactor is a bioreactor in which a cell culture carrier flows by a stirring blade. The culture vessel may be freely selected according to the purpose, from seeding and seed scale to production scale. However, from the viewpoint of scale-up and promoting adhesion and proliferation of cells on the cell culture carrier, the culture vessel is preferably a heterogeneous type bioreactor. However, the cell culture carrier may be placed in a homogeneous type bioreactor and used as the culture vessel of the present invention.

[Culturing Step in First Production Method]

The conditions in the culturing step may be appropriately selected according to the type of the raw material cell or the culture vessel from the conditions usually used. For example, the cells can be cultured at a temperature of 37°C, a humidity of 95%, and a $CO_2$ concentration of 5%. The $CO_2$ concentration may be 1 to 10% or 3 to 8%.

[Detachment Step in First Production Method]

In the detachment step, the cultured cells are detached from the hydrogel by a liquid flow. While the hydrogel in the present invention has excellent cell adhesiveness that sufficiently functions as a cell culture carrier even when the culture vessel is a bioreactor, cells that have proliferated on the surface thereof can be satisfactorily detached by the liquid flow without using a proteolytic enzyme. In addition, unlike the method for recovering cells cultured on the cell culture carrier by dissolving the cell culture carrier, the dissolved cell culture carrier does not become mixed into the recovered cells. Furthermore, unlike the method that involve changing the temperature of the composition contained in the culture vessel for detachment, the cells can be detached rapidly while maintaining their activity. Therefore, the method of the present invention enables the stable production of cells while maintaining their good activity.

The reason why the hydrogel in the present invention has excellent cell adhesiveness and the cells that have proliferated on the hydrogel surface can be satisfactorily detached by a liquid flow without using a proteolytic enzyme is not clear, but the following reasons are considered. However, the following reason is not intended to limit the above reason in the present invention. The crosslinked body of the vinyl alcohol-based polymer contained in the hydrogel has a part exhibiting low cell adhesiveness due to a hydroxy group and a part exhibiting high cell adhesiveness due to an introduced functional group (for example, an amino group). In addition, the cell adhesion factor preferably contained in the hydrogel exhibits high cell adhesiveness. As described above, since a part exhibiting high cell adhesiveness and a part exhibiting low cell adhesiveness are mixed in the hydrogel in the present invention in a wellbalanced manner, it is considered that the hydrogel in the present invention has an appropriate cell adhesiveness that allows cell detachment by a liquid flow, while being sufficiently robust for cell culture.

From the viewpoint of maintaining the activity of the cell, the detachment step is preferably performed under an environment similar to the cell culture conditions, for example, under conditions of a temperature of 37°C, a humidity of 95%, and a $CO_2$ concentration of 5%. However, it is permissible for the temperature, humidity, and/or $CO_2$ concentration to exceed or fall below the above-mentioned levels for a short period of time, as long as such deviation does not reduce the activity of the cells. For example, the $CO_2$ concentration may be under the condition of 1 to 10%.

From the viewpoint of production of cells efficiency, the detachment step is preferably performed after reaching a state of preferably 60 to 100% confluent, more preferably 65 to 100% confluent, and still more preferably 70 to 100% confluent in the culturing step. Here, for example, 60% confluent means a state where cells cover 60% of the surface area of the cell culture carrier.

From the viewpoint of production of cells efficiency, the detachment step is also preferably performed until a state of 0 to 90% confluent, more preferably 0 to 75% confluent, and still more preferably 0 to 60% confluent is reached.

The confluent ratio may be measured, for example, by acquiring a microscopic observation image of a hydrogel harvested from the inside of a culture vessel, and obtaining the area of the carrier surface coated with cells from image analysis.

In one preferable embodiment, the liquid flow is generated by one or more means selected from the group consisting of the following (i) to (iv):

(i) suction and discharge of at least a part of the composition through a tube installed in the culture vessel;
(ii) shaking of the composition;
(iii) stirring of the composition by stirring blades; and
(iv) discharge of a gas and/or a liquid into the culture vessel.

The liquid flow in the present invention causes a flow of liquid having a flow velocity to act on the cells present on the hydrogel surface to apply a shear stress to the cells present on the hydrogel surface, and the cells are detached from the hydrogel surface by the force of the stress. Therefore, a method for applying a liquid flow to which such a shear stress is applied is not particularly limited and may be freely used. In general, when cells are subjected to a shear stress of 5 Pa or more, a significant decrease in activity may be observed or cells may die. Therefore, the liquid flow in the present invention needs to be a liquid flow that gives a shear stress smaller than this. The shear stress applied to the cells greatly varies depending on the shape of the culture vessel and the conditions of liquid flow, shaking, stirring, and discharge. It is possible to determine the shear stress by simulating it through computational fluid dynamics (CFD) calculations.

As the tube in the means (i), any tube may be used as long as at least part of the composition can be sucked and discharged. One or more tubes (hereinafter also referred to as a suction tube) for sucking at least a part of the composition may be installed according to the amount of the composition or the type of culture vessel, and it is preferable that the suction port of the suction tube is disposed below the liquid level of the composition. At least one tube for discharging at least a part of the composition (hereinafter also referred to as a discharge tube) may be installed, either singly or in plurality, depending on the amount of the composition or the type of culture vessel. The suction tube may also serve as a discharge tube, and the suction tube and the discharge tube may be separate tubes. When the suction tube and the discharge tube are separate tubes, the discharge port of the discharge tube may be disposed below the liquid level of the composition, near the liquid level, or above the liquid level. From the viewpoint of the recovery rate of the detached cells, it is preferable that half or more, preferably all of the at least part of the sucked composition is returned into the culture vessel by discharge. The material of the tube, particularly the material of the inner wall of the tube, may be any material that does not adversely affect production of cells. Examples of such materials include glass, stainless steel, polyethylene, polypropylene, polymethyl methacrylate, polystyrene, polycarbonate, polyester, ethylene-vinyl alcohol copolymer, polyamide, polyimide, or combinations of two or more thereof. From the viewpoint of use in the production of cells, the tube is preferably sterilized as with the hydrogel. The shape of the cross section of the tube is not particularly limited, and may be a circle, an ellipse, a triangle, a quadrangle, a star, or a combination of two or more thereof, and may be a cross section including irregularities. The tube may be straight or curved or bent. The cross-sectional area of the tube may be appropriately selected according to the amount of the composition, and may be, for example, 0.001 to 8,000 $cm^2$, preferably 0.002 to 2,000 $cm^2$, more preferably 0.005 to 1,000 $cm^2$, and may be constant or variable over the length direction. Components other than the hydrogel in the composition may be sucked by sucking the liquid from the suction port through the porous material to be described later. The suction or discharge flow velocity is preferably 0.0001 to 100 m/s, more preferably 0.001 to 50 m/s, and still more preferably 0.005 to 30 m/s.

The number of times of suction and discharge is not limited. For example, one cycle of suction and discharge may be repeated a plurality of times, at least a part of the composition may be sucked once and then discharged twice, or conversely, a part of the composition may be sucked twice and then discharged once. Suction and discharge may be performed continuously or intermittently.

In the means (ii), for example, the composition may be shaken by using ultrasonic waves, a vortex mixer, a rotary shaker, a see-saw (wave-form shaking) shaker, a vertical or horizontal shaker, or a combination of two or more thereof. Conditions for the ultrasonic treatment or the vortex operation may be appropriately selected depending on the amount of the composition or the type of culture vessel. The shaking may be performed continuously or intermittently. Also in the shaking, the flow velocity inside the culture vessel is preferably 0.0001 to 100 m/s, more preferably 0.001 to 50 m/s, and still more preferably 0.005 to 30 m/s.

In the means (iii), commonly used stirring blades can be used. Examples of the stirring blade include a paddle blade, a flat paddle blade, a propeller blade, a turbine blade, an elephant ear blade, an anchor blade, a cone blade, a full-zone blade, a ribbon blade, and a screw blade. The dimensions of the stirring blade, the number of paddles or the number of disks, and the stirring conditions may be appropriately selected according to the amount of the composition or the type of culture vessel. The stirring can be performed continuously or intermittently. Also in the shaking, the flow velocity inside the culture vessel is preferably 0.0001 to 100 m/s, more preferably 0.001 to 50 m/s, and still more preferably 0.005 to 30 m/s.

The gas and liquid to be discharged by the means (iv) may be any gas and liquid as long as the gas and liquid do not adversely affect the activity of the cell. Examples of the gas include an inert gas such as air, nitrogen, or argon, or a gas obtained by mixing two or more thereof. Examples of liquids include liquid media (preferably, the same liquid medium as the liquid medium contained in the composition), physiological saline, buffer solutions, or mixtures of two or more thereof. One of gas and liquid may be discharged, or both gas and liquid may be discharged. The discharge may be performed continuously or intermittently. From the viewpoint of generating a sufficient liquid flow, it is preferable that the discharge tube be thin, and when both gas and liquid are to be discharged, it is preferable that the gas discharge tube and the liquid discharge tube be separate tubes. The cross-sectional area of the tube may be appropriately selected, and may be, for example, 0.001 to 10 $cm^2$, preferably 0.002 to 5 $cm^2$, more preferably 0.005 to 1 $cm^2$, and may be constant or variable over the length direction. The tube may be branched in the middle. For example, when nitrogen is discharged from a tube connected to a nitrogen cylinder, the tube may have a plurality of discharge ports. The discharge port of the discharge tube may be disposed below the liquid level of the composition, near the liquid level, or above the liquid level. As such a tube, one similar to the tube described based on the means (i) can be used. The discharge conditions such as the amount of gas and liquid to be discharged may be appropriately selected depending on the amount of the composition or the type of culture vessel. For example, the discharge flow velocity is preferably 0.0001 to 100 m/s, more preferably 0.001 to 50 m/s, and still more preferably 0.005 to 30 m/s.

In one embodiment of the present invention, the production method may include a step of bringing at least a part of the composition in the culture vessel into contact with a solution in which the concentration of the proteolytic enzyme is preferably 1.5 mg/mL or less and the concentration of the chelating agent is preferably 0.01 mg/mL or more, and detaching

EP 4 715 042 A1

the cultured cells from the surface of the hydrogel. The detachment step using the chelating agent may be performed before the detachment step by the liquid flow described above, simultaneously with the detachment step by the liquid flow, and/or after the detachment step by the liquid flow. The detachment step using a chelating agent is preferably performed before the detachment step by the liquid flow or simultaneously with the detachment step by the liquid flow from the viewpoint of enhancing the detachability of cells.

The number of contacts is not limited. For example, the treatment may be performed once before the detachment step by the liquid flow and once after the detachment step by the liquid flow, or may be performed a plurality of times by replacing the solution.

The concentration of a proteolytic enzyme (for example, trypsin) generally used for detaching cells is usually approximately 2.5 mg/mL, and there is a problem that when cells are detached from a cell culture carrier using the proteolytic enzyme, the cells can be damaged. The solution used in the detachment step using the chelating agent in the present invention may contain a proteolytic enzyme as long as cells are not damaged, and the concentration thereof is preferably 1.5 mg/mL or less, more preferably 1.0 mg/mL or less, still more preferably 0.5 mg/mL or less, and particularly preferably 0 mg/mL. The concentration of the proteolytic enzyme in the solution can be measured, for example, by ELISA using an antigen-antibody reaction.

The chelating agent in the present invention plays a role of chelating essential calcium ions and magnesium ions in cell-cell adhesion and adhesion between cells and the cell culture carrier. The concentration of the chelating agent is preferably 0.01 mg/mL or more, more preferably 0.02 mg/mL or more, and still more preferably 0.03 mg/mL or more (for example, 0.05 mg/mL or more, 0.08 mg/mL or more, or 0.10 mg/mL or more). When the concentration of the chelating agent is above the lower limit, the cells can be detached from the hydrogel more rapidly. The upper limit value of the concentration of the chelating agent is usually 15 mg/mL or less, and preferably 1.5 mg/mL or less from the viewpoint of cell viability. The concentration of the chelating agent in the solution can be measured by, for example, a colorimetric method using a metal complex that exhibits color upon chelate formation with copper, cobalt, or the like.

Examples of chelating agents include aminocarboxylic acid-based chelating agents such as ethylenediaminetetraacetic acid (EDTA), ethylene glycol tetraacetic acid (EGTA), nitrilotriacetic acid (NTA), and diethylenetriaminepentaacetic acid (DTPA); phosphate-based chelating agents such as sodium tripolyphosphate, sodium pyrophosphate, and sodium orthophosphate; and phosphonate-based chelating agents such as phosphonobutane tricarboxylic acid, amino tris(methylene phosphonic acid), 1-hydroxyethane-1,1-diphosphonic acid, ethylenediaminetetra(methylene phosphonic acid), and diethylenetriaminepenta(methylene phosphonic acid). The chelating agents can be used alone or in combination of two or more thereof. From the viewpoint of good cell detachability and viability, a chelating agent that forms a chelate with a divalent metal ion is preferable, a chelating agent that forms a chelate with a calcium ion and a magnesium ion is particularly preferable, ethylenediaminetetraacetic acid and ethylene glycol tetraacetic acid are more particularly preferable, and ethylenediaminetetraacetic acid is most preferable. These may be alkali metal salts.

Proteolytic enzymes are enzymes capable of hydrolyzing peptide bonds in proteins. Specific examples include trypsin, chymotrypsin, lysyl endopeptidase, Accutase, collagenase, natural proteases, elastase, pronase, clostripain, papain, Endo Arg-C, Endo Lys-C, and Endo Asn-C. The proteolytic enzyme may be used in the solution alone or in combination of two or more thereof.

Examples of solvents in the solution used in the detachment step using a chelating agent include physiological saline, phosphate-buffered saline, Hanks' balanced salt solution, Earle's balanced salt solution, N-2-hydroxyethylpiperazine-N'-ethanesulfonic acid (HEPES), and mixed solvents of two or more of these.

It is preferable that the solution does not contain calcium ions and magnesium ions.

In this embodiment, at least a part of the composition in the culture vessel, preferably at least 50 mass% of the composition, more preferably at least 75 mass% of the composition, and still more preferably all of the composition in the culture vessel is brought into contact with the solution. Hereinafter, in the present embodiment, "at least a part of the composition in the culture vessel, preferably at least 50 mass% of the composition, more preferably at least 75 mass% of the composition, and still more preferably all of the composition in the culture vessel" is abbreviated as "at least a part of the composition". "Contact" refers to a procedure of an operation in which the composition is brought into contact with the solution. Specific examples of this operation include replacing a liquid component contained in at least a part of the composition with the solution, and mixing at least a part of the composition with the solution.

When the liquid component contained in at least a part of the composition is replaced with the solution, the amount of the replaced liquid component is preferably 20 to 100 mass%, more preferably 40 to 100 mass%, and still more preferably 60 to 100 mass% based on the total mass of the composition. The substitution method is not particularly limited, and substitution may be performed by a known method. For example, the liquid component contained in at least a part of the composition can be replaced with the solution by filtering out at least a part of the liquid component from the composition using a porous material that can be used in the separation step to be described later and mixing the remaining composition and the solution; or by removing at least a part of the liquid component from the upper portion of the culture vessel after precipitating the hydrogel in the composition in the culture vessel, and by mixing the remaining composition and the solution.

21

When at least a part of the composition and the solution are mixed, the amount of the solution to be mixed is preferably 1 to 1,000 mass%, more preferably 5 to 100 mass%, and still more preferably 10 to 50 mass% based on the total mass of the composition. The mixing method is not particularly limited, and mixing may be performed by a known method.

The temperature when at least a part of the composition is brought into contact with the solution is usually 0 to 40°C, and preferably 15 to 37°C, from the viewpoint of cell viability and activity.

The contact time may be appropriately selected depending on the concentration of the chelating agent contained in the solution, the adhesion strength of the cell, and the cell viability. The contact time is usually 1 second to 30 minutes, preferably 1 to 10 minutes, and more preferably 2 to 5 minutes.

Another method for producing cells of the present invention, comprises:

a step of preparing a culture vessel containing a composition comprising a liquid medium, a hydrogel containing a crosslinked body of a vinyl alcohol-based polymer, and raw material cells (preparation step);
a step of culturing the raw material cells on a surface of the hydrogel (culturing step); and
a step of detaching the cultured cells from the surface of the hydrogel by bringing at least a part of the composition in the culture vessel into contact with a solution in which a concentration of a proteolytic enzyme is 1.5 mg/mL or less and a concentration of a chelating agent is 0.01 mg/mL or more (detachment step). Hereinafter, this production method is also referred to as a "second production method".

The descriptions described in the paragraphs [Preparation Step in First Production Method] and [Culturing Step in First Production Method] in the first production method can be applied to the preparation step and the culturing step in the second production method.

[Detachment Step in Second Production Method]

In the detachment step, at least a part of the composition in the culture vessel is brought into contact with a solution in which the concentration of the proteolytic enzyme is preferably 1.5 mg/mL or less and the concentration of the chelating agent is preferably 0.01 mg/mL or more, and detaching the cultured cells from the surface of the hydrogel. In one embodiment of the present invention, the solution is preferably a solution that does not contain a proteolytic enzyme and contains 0.01 mg/mL or more of a chelating agent. The number of contacts is not limited and may be performed one or more times.

The concentration of a proteolytic enzyme (for example, trypsin) generally used for detaching cells is usually approximately 2.5 mg/mL, and there is a problem that when cells are detached from a cell culture carrier using the proteolytic enzyme, the cells can be damaged. The solution used in the detachment step using the chelating agent in the present invention may contain a proteolytic enzyme as long as cells are not damaged, and the concentration thereof is 1.5 mg/mL or less, preferably 1.0 mg/mL or less, more preferably 0.5 mg/mL or less, and particularly preferably 0 mg/mL. The concentration of the proteolytic enzyme in the solution can be measured, for example, by ELISA using an antigen-antibody reaction.

While the hydrogel in the present invention has excellent cell adhesiveness that sufficiently functions as a cell culture carrier even when the culture vessel is a bioreactor, the cells that have proliferated on the surface can be satisfactorily detached using the chelating agent-containing solution described above even without or with only a low concentration of proteolytic enzyme. In addition, unlike the method for recovering cells cultured on the cell culture carrier by dissolving the cell culture carrier, the dissolved cell culture carrier does not become mixed into the recovered cells. Furthermore, unlike the method for varying the temperature of the composition contained in the culture vessel for detachment, the cells can be detached rapidly while maintaining their activity. Therefore, by the method of the present invention, it is possible to stably produce cells of which activity remains well maintained.

The reason why the hydrogel according to the present invention, while having excellent cell adhesiveness, allows cells that have proliferated on the surface to be favorably detached using the chelating agent-containing solution even without or with only a low concentration of proteolytic enzyme is not clear, but the following reasons are considered. However, the following reason is not intended to limit the above reason in the present invention. The crosslinked body of the vinyl alcohol-based polymer contained in the hydrogel has a part exhibiting low cell adhesiveness due to a hydroxy group and a part exhibiting high cell adhesiveness due to an introduced functional group (for example, an amino group). In addition, the cell adhesion factor preferably contained in the hydrogel exhibits high cell adhesiveness. As described above, since a part exhibiting high cell adhesiveness and a part exhibiting low cell adhesiveness are mixed in the hydrogel in the present invention in a well-balanced manner, it is considered that the hydrogel in the present invention has an appropriate cell adhesiveness that allows cell detachment by the action of the chelating agent-containing solution, while being sufficiently robust for cell culture.

From the viewpoint of maintaining the activity of the cell, the detachment step is preferably performed under an environment similar to the cell culture conditions, for example, under conditions of a temperature of 37°C, a humidity of

95%, and a $CO_2$ concentration of 5%. However, it is permissible for the temperature, humidity, and/or $CO_2$ concentration to exceed or fall below the above-mentioned levels for a short period of time, as long as such deviation does not reduce the activity of the cells. For example, the $CO_2$ concentration may be under the condition of 1 to 10%.

From the viewpoint of production of cells efficiency, the detachment step is preferably performed after reaching a state of preferably 60 to 100% confluent, more preferably 65 to 100% confluent, and still more preferably 70 to 100% confluent in the culturing step. Here, for example, 60% confluent means a state where cells cover 60% of the surface area of the cell culture carrier.

From the viewpoint of production of cells efficiency, the detachment step is also preferably performed until a state of 0 to 90% confluent, more preferably 0 to 75% confluent, and still more preferably 0 to 60% confluent is reached.

The confluent ratio can be measured, for example, by acquiring a microscopic observation image of a hydrogel harvested from the inside of a culture vessel, and obtaining the area of the carrier surface coated with cells from image analysis.

The chelating agent in the present invention plays a role of chelating essential calcium ions and magnesium ions in cell-cell adhesion and adhesion between cells and the cell culture carrier. The concentration of the chelating agent is 0.01 mg/mL or more, preferably 0.02 mg/mL or more, and more preferably 0.03 mg/mL or more (for example, 0.05 mg/mL or more, 0.08 mg/mL or more, or 0.10 mg/mL or more). When the concentration of the chelating agent is above the lower limit, the cells can be detached from the hydrogel more rapidly. The upper limit value of the concentration of the chelating agent is usually 15 mg/mL or less, and preferably 1.5 mg/mL or less from the viewpoint of cell viability. The concentration of the chelating agent in the solution can be measured by, for example, a colorimetric method using a metal complex that exhibits color upon chelate formation with copper, cobalt, or the like.

Examples of chelating agents include aminocarboxylic acid-based chelating agents such as ethylenediaminetetraacetic acid (EDTA), ethylene glycol tetraacetic acid (EGTA), nitrilotriacetic acid (NTA), and diethylenetriaminepentaacetic acid (DTPA); phosphate-based chelating agents such as sodium tripolyphosphate, sodium pyrophosphate, and sodium orthophosphate; and phosphonate-based chelating agents such as phosphonobutane tricarboxylic acid, amino tris(methylene phosphonic acid), 1-hydroxyethane-1,1-diphosphonic acid, ethylenediaminetetra(methylene phosphonic acid), and diethylenetriaminepenta(methylene phosphonic acid). The chelating agents can be used alone or in combination of two or more thereof. From the viewpoint of good cell detachability and viability, a chelating agent that forms a chelate with a divalent metal ion is preferable, a chelating agent that forms a chelate with a calcium ion and a magnesium ion is particularly preferable, ethylenediaminetetraacetic acid and ethylene glycol tetraacetic acid are more particularly preferable, and ethylenediaminetetraacetic acid is most preferable. These may be alkali metal salts.

Proteolytic enzymes are enzymes capable of hydrolyzing peptide bonds in proteins. Specific examples include trypsin, chymotrypsin, lysyl endopeptidase, Accutase, collagenase, natural proteases, elastase, pronase, clostripain, papain, Endo Arg-C, Endo Lys-C, and Endo Asn-C. The proteolytic enzyme may be included in the solution alone or in combination of two or more.

Examples of solvents in the solution used in the detachment step using a chelating agent include physiological saline, phosphate-buffered saline, Hanks' balanced salt solution, Earle's balanced salt solution, N-2-hydroxyethylpiperazine-N'-ethanesulfonic acid (HEPES), and mixed solvents of two or more of these.

It is preferable that the solution does not contain calcium ions and magnesium ions.

In this embodiment, at least a part of the composition in the culture vessel, preferably at least 50 mass% of the composition, more preferably at least 75 mass% of the composition, and still more preferably all of the composition in the culture vessel is brought into contact with the solution. Hereinafter, in the present embodiment, "at least a part of the composition in the culture vessel, preferably at least 50 mass% of the composition, more preferably at least 75 mass% of the composition, and still more preferably all of the composition in the culture vessel" is abbreviated as "at least a part of the composition". "Contact" refers to a procedure of an operation in which the composition is brought into contact with the solution. Specific examples of this operation include replacing a liquid component contained in at least a part of the composition with the solution, and mixing at least a part of the composition with the solution.

When the liquid component contained in at least a part of the composition is replaced with the solution, the amount of the replaced liquid component is preferably 20 to 100 mass%, more preferably 40 to 100 mass%, and still more preferably 60 to 100 mass% based on the total mass of the composition. The substitution method is not particularly limited, and substitution may be performed by a known method. For example, the liquid component contained in at least a part of the composition can be replaced with the solution by filtering out at least a part of the liquid component from the composition using a porous material that may be used in the separation step to be described later and mixing the remaining composition and the solution; or by removing at least a part of the liquid component from the upper portion of the culture vessel after precipitating the hydrogel in the composition in the culture vessel, and by mixing the remaining composition and the solution.

When at least a part of the composition and the solution are mixed, the amount of the solution to be mixed is preferably 1 to 1,000 mass%, more preferably 5 to 100 mass%, and still more preferably 10 to 50 mass% based on the total mass of the composition. The mixing method is not particularly limited, and mixing can be performed by a known method.

The temperature when at least a part of the composition is brought into contact with the solution is usually 0 to 40°C, and preferably 15 to 37°C, from the viewpoint of cell viability and activity.

The contact time may be appropriately selected according to the concentration of the chelating agent contained in the solution, the adhesion strength of the cell, and the cell viability. The contact time is usually 1 second to 30 minutes, preferably 1 to 10 minutes, and more preferably 2 to 5 minutes.

In one embodiment of the present invention, the production method may further include a step of detaching the cultured cells from the surface of the hydrogel by a liquid flow. The detachment step by the liquid flow may be performed before the detachment step by the action of the chelating agent-containing solution, simultaneously with the detachment step by the action of the chelating agent-containing solution, and/or after the detachment step by the action of the chelating agent-containing solution. From the viewpoint of enhancing the detachability of cells, it is preferable that the detaching step by the liquid flow be performed after the detachment step using a chelating agent or simultaneously with the detaching step using a chelating agent.

The number of detachment steps by the liquid flow is not limited. For example, the detachment step may be performed once before the detachment step by the action of the chelating agent-containing solution and once after the detachment step by the action of the chelating agent-containing solution, or may be performed a plurality of times after the detachment step using the chelating agent.

In one preferable embodiment, the liquid flow is generated by one or more means selected from the group consisting of the following (i) to (iv):

(i) suction and discharge of at least a part of the composition through a tube installed in the culture vessel;
(ii) shaking of the composition;
(iii) stirring of the composition by stirring blades; and
(iv) discharge of a gas and/or a liquid into the culture vessel.

The liquid flow in the present invention causes a flow of liquid having a flow velocity to act on the cells present on the hydrogel surface to apply a shear stress to the cells present on the hydrogel surface, and the cells are detached from the hydrogel surface by the force of the stress. Therefore, a method for applying a liquid flow to which such a shear stress is applied is not particularly limited and may be freely used. In general, when cells are subjected to a shear stress of 5 Pa or more, a significant decrease in activity may be observed or cells may die. Therefore, the liquid flow in the present invention needs to be a liquid flow that gives a shear stress smaller than this. The shear stress applied to the cells greatly varies depending on the shape of the culture vessel and the conditions of liquid flow, shaking, stirring, and discharge. It is possible to determine the shear stress by simulating it through computational fluid dynamics (CFD) calculations.

As the tube in the means (i), any tube may be used as long as at least part of the composition can be sucked and discharged. One or more tubes (hereinafter also referred to as a suction tube) for sucking at least a part of the composition may be installed according to the amount of the composition or the type of culture vessel, and it is preferable that the suction port of the suction tube is disposed below the liquid level of the composition. At least one tube for discharging at least a part of the composition (hereinafter also referred to as a discharge tube) may be installed, either singly or in plurality, depending on the amount of the composition or the type of culture vessel. The suction tube may also serve as a discharge tube, and the suction tube and the discharge tube may be separate tubes. When the suction tube and the discharge tube are separate tubes, the discharge port of the discharge tube may be disposed below the liquid level of the composition, near the liquid level, or above the liquid level. From the viewpoint of the recovery rate of the detached cells, it is preferable that half or more, preferably all of the at least part of the sucked composition is returned into the culture vessel by discharge. The material of the tube, particularly the material of the inner wall of the tube, is not limited as long as the material does not adversely affect production of cells. Examples thereof include glass, stainless steel, polyethylene, polypropylene, polymethyl methacrylate, polystyrene, polycarbonate, polyester, ethylene-vinyl alcohol copolymer, polyamide, polyimide, or combinations of two or more thereof. From the viewpoint of use in the production of cells, the tube is preferably sterilized as with the hydrogel. The shape of the cross section of the tube is not particularly limited, and may be a circle, an ellipse, a triangle, a quadrangle, a star, or a combination of two or more thereof, and may be a cross section including irregularities. The tube may be straight or curved or bent. The cross-sectional area of the tube may be appropriately selected according to the amount of the composition, and may be, for example, 0.001 to 8,000 $cm^2$, preferably 0.002 to 2,000 $cm^2$, more preferably 0.005 to 1,000 $cm^2$, and may be constant or variable over the length direction. Components other than the hydrogel in the composition may be sucked by sucking the liquid from the suction port through the porous material to be described later. The suction or discharge flow velocity is preferably 0.0001 to 100 m/s, more preferably 0.001 to 50 m/s, and still more preferably 0.005 to 30 m/s.

The number of times of suction and discharge is not limited. For example, one cycle of suction and discharge may be repeated a plurality of times, at least a part of the composition may be sucked once and then discharged twice, or conversely, a part of the composition may be sucked twice and then discharged once. Suction and discharge may be performed continuously or intermittently.

In the means (ii), for example, the composition can be shaken by using ultrasonic waves, a vortex mixer, a rotary shaker, a see-saw (wave-form shaking) shaker, a vertical or horizontal shaker, or a combination of two or more thereof. Conditions for the ultrasonic treatment or the vortex operation may be appropriately selected depending on the amount of the composition or the type of culture vessel. The shaking can be performed continuously or intermittently. Also in the shaking, the flow velocity inside the culture vessel is preferably 0.0001 to 100 m/s, more preferably 0.001 to 50 m/s, and still more preferably 0.005 to 30 m/s.

In the means (iii), commonly used stirring blades may be used. Examples of the stirring blade include a paddle blade, a flat paddle blade, a propeller blade, a turbine blade, an elephant ear blade, an anchor blade, a cone blade, a full-zone blade, a ribbon blade, and a screw blade. The dimensions of the stirring blade, the number of paddles or the number of disks, and the stirring conditions may be appropriately selected according to the amount of the composition or the type of culture vessel. The stirring can be performed continuously or intermittently. Also in the shaking, the flow velocity inside the culture vessel is preferably 0.0001 to 100 m/s, more preferably 0.001 to 50 m/s, and still more preferably 0.005 to 30 m/s.

The gas and liquid to be discharged by the means (iv) may be any gas and liquid as long as the gas and liquid do not adversely affect the activity of the cell. Examples of the gas include an inert gas such as air, nitrogen, or argon, or a gas obtained by mixing two or more thereof. Examples of liquids include liquid media (preferably, the same liquid medium as the liquid medium contained in the composition), physiological saline, buffer solutions, or mixtures of two or more thereof. One of gas and liquid may be discharged, or both gas and liquid may be discharged. The discharge may be performed continuously or intermittently. From the viewpoint of generating a sufficient liquid flow, it is preferable that the discharge tube be thin, and when both gas and liquid are to be discharged, it is preferable that the gas discharge tube and the liquid discharge tube be separate tubes. The cross-sectional area of the tube may be appropriately selected, and may be, for example, 0.001 to 10 cm$^2$, preferably 0.002 to 5 cm$^2$, more preferably 0.005 to 1 cm$^2$, and may be constant or variable over the length direction. The tube may be branched in the middle. For example, when nitrogen is discharged from a tube connected to a nitrogen cylinder, the tube may have a plurality of discharge ports. The discharge port of the discharge tube may be disposed below the liquid level of the composition, near the liquid level, or above the liquid level. As such a tube, one similar to the tube described based on the means (i) can be used. The discharge conditions such as the amount of gas and liquid to be discharged may be appropriately selected depending on the amount of the composition or the type of culture vessel. For example, the discharge flow velocity is preferably 0.0001 to 100 m/s, more preferably 0.001 to 50 m/s, and still more preferably 0.005 to 30 m/s.

(Separation Step)

In one embodiment, the production method of the present invention (the first production method and the second production method) further includes a step of separating the detached cells and the hydrogel (separation step). By the separation, the produced cells and the hydrogel from which the cells have been detached can be respectively recovered.

In one preferable embodiment, the detached cells and the hydrogel are separated using a porous material. This separation corresponds to filtration separation. That is, the hydrogel does not pass through the porous material, and the liquid containing the detached cells passes through the porous material. Due to the volume of the hydrogel which is significantly larger compared to the volume of the detached cells, the detached cells and the hydrogel can be easily and rapidly separated. The easy and rapid separation may not only improve production of cells efficiency, but also result in better maintenance of the activity of the produced cells, thereby achieving more stable production of the cells. The porous material may be any material as long as the material can filter and separate the detached cells and the hydrogel, and examples thereof include a mesh formed by weaving fibers in a lattice shape, a porous sheet, and a punched metal.

The material of the mesh is not particularly limited as long as the material does not adversely affect the production of cells, and may be, for example, chemical fibers such as nylon, polyester, polypropylene, and polyethylene, metal wires such as stainless steel, aluminum, copper, brass, and titanium, or any combination thereof. The shape of the mesh eye is not particularly limited as long as the detached cells and the hydrogel can be filtered and separated, and may be, for example, a lattice shape, a polygonal shape, or a combination of two or more thereof. The mesh opening size is not particularly limited as long as the detached cells and the hydrogel can be filtered and separated, and may be appropriately selected according to the particle diameter of the hydrogel. Since the diameter of adherent cells is generally 10 to 30 $\mu$m, for example, when using a hydrogel having an average particle diameter of 180 to 400 $\mu$m in an equilibrium swollen state in phosphate-buffered saline, separation can be easily performed by using a mesh with an opening size of 100 $\mu$m. Since the hydrogel of the present invention easily swells upon contact with a liquid such as a liquid medium, the hydrogel is in an equilibrium swollen state at the stage of the separation step.

The material of the porous sheet is not particularly limited as long as the material does not adversely affect the production of cells. For example, the porous sheet may be a sheet of a synthetic resin such as nylon, polyester, polypropylene, or polyethylene, or a sheet of a metal such as stainless steel, aluminum, copper, brass, or titanium. The thickness of the porous sheet is also not particularly limited, and may be appropriately selected according to the material and/or mechanical strength of the porous sheet. The thickness of the porous sheet is usually 0.01 mm to 5 mm, and preferably 0.05 mm to 0.5 mm. The shape of the holes is not particularly limited as long as the detached cells and the hydrogel can be filtered and separated, and may be, for example, circular, elliptical, polygonal, slit-shaped, irregular, or a combination of two or more thereof. The dimensions of the holes are not particularly limited as long as the detached cells and the hydrogel can be filtered and separated, and may be appropriately selected according to the particle diameter of the hydrogel. The dimensions of each hole may be the same or different. For example, when using a hydrogel having an average particle diameter of 180 to 400 $\mu$m in an equilibrium swollen state in phosphate-buffered saline, separation can be easily performed by using a porous sheet provided with a large number of circular holes having a diameter of 100 $\mu$m.

The material of the punched metal is not particularly limited as long as the material does not adversely affect the production of cells. For example, the material may be a punched metal such as stainless steel, aluminum, copper, brass, or titanium. The thickness, the shape of the hole, and the dimension of the hole of the punched metal are not particularly limited, and may be equivalent to those of the porous sheet.

In another preferable embodiment, the detached cells and the hydrogel are separated by a difference in sedimentation velocity. The sedimentation velocity in the present invention refers to the velocity at which a solid settling in a liquid reaches a constant speed under drag force, and is also referred to as terminal velocity, final velocity, or terminal sedimentation velocity. The sedimentation velocity v can be calculated from the following equation.

$$v = \sqrt{\frac{4(\rho_p - \rho)gD_p}{3\rho Cd}}$$

$\rho_p$: Density of solid [kg/m$^3$]
$\rho$: Density of liquid [kg/m$^3$]
g:

Gravitational acceleration = 981 [cm/s$^2$]

$D_p$: Diameter of solid [cm]
Cd: Resistance coefficient

For example, when the diameter of the hydrogel according to the present invention in an equilibrium swollen state in phosphate-buffered saline is 300 $\mu$m (that is, 0.03 cm), the density thereof is approximately 1,017 kg/m$^3$, and the resistance coefficient thereof is approximately 96.0. Assuming that the density of the liquid is 1,000 kg/m$^3$, when calculating the sedimentation velocity of the hydrogel based on the above equation, the value is approximately 0.083 cm/s. Meanwhile, the density of cells having a diameter of 20 $\mu$m is approximately 1,050 kg/m$^3$, the resistance coefficient is approximately 15.6 x 10$^5$, and the sedimentation velocity is calculated to be approximately 2.9 x 10$^{-4}$ cm/s. That is, the sedimentation velocity of the hydrogel and the sedimentation velocity of the cells are significantly different. For this reason, when the culture vessel is allowed to stand for a certain period of time after the detachment step, the hydrogel settles at the bottom of the culture vessel, and the cells can be brought into a state of being suspended in the culture vessel. By sucking the liquid from the liquid level in the culture vessel in this state, the liquid containing cells can be recovered and separated from the hydrogel. By sucking the liquid through the porous material described above, it is also possible to further reduce mixing of the hydrogel into the recovered liquid.

Optionally, by washing the separated hydrogel, cells adhering to the hydrogel surface or existing in the vicinity of the hydrogel can be recovered. For example, the separated hydrogel is mixed with a washing liquid such as water or a buffer solution, and the separation step described above is performed again, whereby the cells can be recovered. The amount of the washing liquid, the mixing method or conditions, and the like are not particularly limited, and may be appropriately selected. In addition, when this washing step is performed, the number of times is not limited, and may be one time or a plurality of times.

Thereafter, the cells produced by the method of the present invention can be recovered by subjecting the liquid containing the cells to a conventionally known method such as centrifugation or the use of a cell fractionation filter.

[Reuse of Hydrogel]

The hydrogel from the surface of which the cells have been detached, in the first production method, by using a liquid flow and, as needed, a chelating agent-containing solution, or in the second production method, by using a chelating agent-containing solution and, as needed, a liquid flow, can be recovered and reused for culturing new raw material cells since the cell adhesion function is maintained. That is, the hydrogel separated in the separation step can be reused as a cell culture carrier in the production of cells. Therefore, the present invention also relates to a method for producing cells, comprising: a step of recovering the hydrogel separated in the separation step described above (hydrogel recovery step); a step of preparing a culture vessel containing a composition comprising a liquid medium, the recovered hydrogel, and raw material cells (repeated preparation step); and a step of culturing the raw material cells on the surface of the recovered hydrogel (repeated culturing step).

The hydrogel recovered in the hydrogel recovery step may be washed with water, or a buffer solution, and then used in the repeated preparation step. The drying step and/or the sterilization step as described above may also be performed on the recovered hydrogel or the washed hydrogel.

The description of the [Preparation Step in First Production Method] and the [Culturing Step in First Production Method] described above may be applied to the repeated preparation step and the repeated culturing step. Following the repeated culturing step, a step similar to the [Detachment Step in First Production Method] or the [Detachment Step in Second Production Method] and (Separation Step) described above may be performed. In the repeated detachment step, the same detachment step as the previous detachment step may be adopted, or a different detachment step may be adopted. For example, the detachment step of the first production method may be adopted in both the previous detachment step and the repeated detachment step, the detachment step of the first production method may be adopted in the previous detachment step, the detachment step of the second production method may be adopted in the repeated detachment step, the detachment step of the second production method may be adopted in the previous detachment step (without departing from the gist of the present invention), and the cell detachment step (different from the detachment step in the first production method and the detachment step in the second production method) may be adopted in the repeated detachment step. Thereafter, the hydrogel that has undergone the (separation step) can be further reused as a cell culture carrier.

EXAMPLES

Hereinafter, the present invention will be described in more detail with reference to Examples, but the present invention is not limited to these Examples at all. Physical properties in Examples and Comparative Examples were measured or evaluated by the following procedure.

<Average Particle Diameter of Hydrogel>

The hydrogel in a dry state (approximately 20 mg) and phosphate-buffered saline (approximately 5 mL) to be described later were mixed and swollen at room temperature for 2 hours to prepare a mixed solution.

The hydrogel in a dry state (approximately 20 mg) and the same liquid medium (approximately 5 mL) as the liquid medium contained in the composition were mixed and swollen at room temperature for 2 hours to prepare a mixed solution.

Each of the prepared mixed solutions was charged into a laser diffraction/scattering type particle diameter distribution measuring apparatus (device name: Partica LA-950, manufactured by HORIBA, Ltd.), and the average particle diameter of the hydrogel was measured on a volume basis.

<Introduction Rate of Ethylenically Unsaturated Group and succinyl methylene group>

The introduction rate of an ethylenically unsaturated group of a vinyl alcohol-based polymer having an ethylenically unsaturated group obtained in Synthesis Example described later was measured by $^1$H-NMR. Specifically, the introduction rate of the ethylenically unsaturated group was determined from the ratio of the integral value of the signal of the ethylenically unsaturated group and the signal of the vinyl alcohol-based polymer.

Similarly, $^1$H-NMR measurement could be performed on the crosslinked body of the vinyl alcohol-based polymer in a state of being swollen in a heavy solvent (heavy water), and the introduction rate of the succinyl methylene group was determined from the ratio of the integral value of the signal of the succinyl methylene group and the signal of the crosslinked body of the vinyl alcohol-based polymer.

[$^1$H-NMR Measurement Conditions]

Apparatus: Nuclear magnetic resonance apparatus "JNM-ECX400" manufactured by JEOL Ltd.

Temperature:      25°C

<Degree of Swelling>

The hydrogel in a dry state was allowed to stand at room temperature and reduced pressure overnight, approximately 10 mg ($W_{dry}$) of the hydrogel was then weighed, immersed in 5 mL of phosphate-buffered saline to be described later, and allowed to stand for 30 minutes to swell. The swollen hydrogel was placed on a nylon mesh having an opening size of 100 $\mu$m, a paper sheet was applied from the side of the nylon mesh to suck phosphate-buffered saline, and then the mass ($W_{wet}$) of the hydrogel was measured. The value of ($W_{wet}/W_{dry}$) x 100 (%) was calculated. The same operation was repeated 3 times, and the average value of the calculated ($W_{wet}/W_{dry}$) x 100 (%) values was taken as the degree of swelling.

[Production Example]

[Raw Materials Used]

The main components used in Production Examples are shown below.

<Raw Material PVA>

- PVA28-98: Polyvinyl alcohol (trade name "PVA28-98", viscosity-average degree of polymerization: 1,700; degree of saponification: approximately 98.0 to 99.0 mol%; viscosity (4 mass%, 20°C): 25.0 to 31.0 mPa·s, manufactured by Kuraray Co., Ltd.)

The viscosity-average degree of polymerization of the raw material PVA was measured in accordance with JIS K 6726:1994.

<Ethylenically Unsaturated Group-Containing Compound>

- Vinyl methacrylate: Manufactured by Tokyo Chemical Industry Co., Ltd.

<Synthesis Reagent>

- Triethylamine: Manufactured by FUJIFILM Wako Pure Chemical Corporation
- Succinic anhydride: Manufactured by FUJIFILM Wako Pure Chemical Corporation
- Liquid paraffin: Manufactured by FUJIFILM Wako Pure Chemical Corporation
- Span80: Manufactured by FUJIFILM Wako Pure Chemical Corporation

<Radical Polymerization Initiator>

- Potassium persulfate: Manufactured by FUJIFILM Wako Pure Chemical Corporation

<Complexing Reagent>

- 1-(3-Dimethylaminopropyl)-3-ethylcarbodiimide hydrochloride: Manufactured by FUJIFILM Wako Pure Chemical Corporation
- N-Hydroxysuccinimide:Manufactured by Tokyo Chemical Industry Co., Ltd.

<Cell Adhesion Factor>

- Collagen (Cell matrix, Type I-C): manufactured by Nitta Gelatin Inc.

<Solvent>

- Dimethyl sulfoxide: Manufactured by FUJIFILM Wako Pure Chemical Corporation
- Ion-exchanged water: Ion-exchanged water having an electrical conductivity of $0.08 \times 10^{-4}$ S/m or less
- Phosphate-buffered saline (PBS): Prepared by dissolving PBS tablets (manufactured by Takara Bio Inc.) in a specified amount of ion-exchanged water.
- MES buffer solution: A 0.1 mol/L aqueous solution of 2-morpholinoethanesulfonic acid monohydrate (manufactured by Dojindo Laboratories Co., Ltd.) was prepared and neutralized with a 0.1 mol/L aqueous solution of NaOH to adjust the pH to 5.6.

[Synthesis Example 1]

<Synthesis of Vinyl Alcohol-Based Polymer Having Ethylenically Unsaturated Group>

40 g (structural units: 908 mmol) of PVA28-98 (raw material PVA) was placed in a 1 L separable flask equipped with a Dimroth condenser, 350 mL of dimethyl sulfoxide (DMSO) was added thereto, and stirring of the contents was started using a mechanical stirrer. The contents were heated to 80°C by a water bath and stirred at 80°C for 4 hours. After visually confirming that the raw material PVA was dissolved, 2.1 g (18.7 mmol) of vinyl methacrylate was added to the content under stirring at 80°C, and the mixture was further stirred at 80°C for 3 hours. After cooling, the obtained reaction solution was poured into 2 L of methanol under stirring. Stirring was stopped, and the mixture was allowed to stand for 1 hour. The obtained solid was recovered and washed by immersing the recovered solid in 1 L of methanol for 1 hour and recovering. This washing operation was performed three times in total. The washed solid was vacuum-dried at room temperature overnight to obtain methacryloylated PVA (hereinafter abbreviated as "MA-PVA"). The introduction rate of an ethylenically unsaturated group (methacryloyloxy group) of MA-PVA was 2.0 mol% based on all structural units constituting the raw material PVA. In addition, the viscosity-average degree of polymerization measured according to JIS K 6726:1994 of MA-PVA is 1,700, which is the same as that of the raw material PVA.

<Preparation of Dried MA-PVA Gel Spherical Particles>

440 mL of ion-exchanged water was added to 60 g of MA-PVA, and the mixture was stirred at 80°C for 4 hours to dissolve the MA-PVA. The obtained MA-PVA aqueous solution was cooled to room temperature, and potassium persulfate as a water-soluble thermal radical polymerization initiator was added to and dissolved in the aqueous solution to be 0.1 mass%, thereby preparing an uncrosslinked polymer solution.

3,300 mL of liquid paraffin and 8 g of Span 80 were placed in a 5 L separable flask equipped with a Dimroth condenser, and the uncrosslinked polymer solution was slowly added thereto. The obtained mixed solution was stirred at 350 rpm using a mechanical stirrer to obtain a W/O dispersion. The W/O dispersion was heated to 40 °C using a water bath, and the inside of the separable flask was purged with nitrogen for 30 minutes. Thereafter, stirring was continued at 70°C for 3 hours. After cooling to room temperature, the obtained liquid paraffin in which spherical particles of MA-PVA gel were dispersed was filtered through a mesh with an opening size of 100 μm. The filtered MA-PVA gel spherical particles were washed with a total of 3 L of hexane to remove the liquid paraffin, and then classified using a JIS standard sieve to obtain MA-PVA gel spherical particles having a particle diameter of 180 to 300 μm. The gel spherical particles were put into 1 L of acetone, dehydrated, and dried under reduced pressure to obtain dried MA-PVA gel spherical particles (hereinafter abbreviated as "dried MA-PVA gel spherical particles").

<Introduction of Succinic Acid into Dried MA-PVA Gel Spherical Particles>

1.5 g of dried MA-PVA gel spherical particles (structural units: 33 mmol) were placed in a 0.5 L separable flask equipped with a Dimroth condenser, 100 mL of dimethyl sulfoxide (DMSO) was added thereto, and the contents were stirred using a mechanical stirrer to swell the dried MA-PVA gel spherical particles. The content was stirred at 60°C for 1 hour using a water bath, then 364 mg (3.6 mmol) of triethylamine and 330 mg (3.3 mmol) of succinic anhydride were added thereto, and the mixture was further stirred at 60°C for 5 hours. After the cooling, the gel spherical particles into which succinic acid was introduced were filtered and washed twice with 100 mL of DMSO and twice with 100 mL of ion-exchanged water. The washed gel spherical particles were immersed in 100 mL of acetone and recovered to be dehydrated. This dehydration operation was performed three times in total. The dehydrated gel spherical particles were dried under reduced pressure to obtain dried MA-PVA gel spherical particles (hereinafter abbreviated as "dried SA-modified MA-PVA gel spherical particles") into which succinic acid was introduced. The introduction rate of the succinyl methylene group was 10.9 mol% based on all structural units constituting the MA-PVA.

<Complexing with Collagen>

1 g of dried SA-modified MA-PVA gel spherical particles (succinic acid introduction amount: approximately 1.9 mmol) were swollen in MES buffer solution overnight at room temperature. The swollen gel spherical particles were dispersed in 45 mL of MES buffer solution, and while stirring with a magnetic stirrer, 0.78 g (6.8 mmol) of N-hydroxysuccinimide and 0.65 g (3.4 mmol) of 1-(3-dimethylaminopropyl)-3-ethylcarbodiimide hydrochloride were added to the dispersion and shaken at room temperature for 1 hour. The gel spherical particles recovered from the dispersion were washed by immersing the gel spherical particles in 30 mL of a MES buffer solution for 10 minutes to recover the gel spherical particles. This washing operation was repeated three times. The washed gel spherical particles were added to 70 mL of 1 mg/mL collagen MES buffer solution. The obtained mixture was shaken at room temperature for 3 hours, and the gel spherical particles recovered from the mixture were washed by immersing in 70 mL of ion-exchanged water (warmed to 60°C) for 20 minutes to recover the gel spherical particles. This washing operation was repeated twice to obtain SA-MA-PVA gel spherical particles in which collagen was complexed (hereinafter abbreviated as "collagen-SA-modified MA-PVA gel spherical particles").

A part of the collagen-SA-modified MA-PVA gel spherical particles was immersed overnight in an excess of PBS, and the amount of immobilized collagen per 100 mg of gel mass was measured by the bicinchoninic acid (BCA) method (BCA Protein Assay Kit, manufactured by Takara Bio Inc.), resulting in 10.5 µg/100 mg of gel spherical particles.

The remaining collagen-SA-modified MA-PVA gel spherical particles swollen in ion-exchanged water were dehydrated by immersing in 50 mL of ethanol to recover the gel spherical particles. This dehydration operation was repeated three times, and then vacuum drying was performed at room temperature overnight to obtain dried collagen-SA-modified MA-PVA gel spherical particles (hereinafter abbreviated as "hydrogel (1)").

Next, the average particle diameter and the degree of swelling of the hydrogel (1) were measured. The hydrogel (1) in a swollen state in phosphate-buffered saline had an average particle diameter of 350 µm and a degree of swelling of 1010%. The average particle diameter of hydrogel (1) in a swollen state in DMEM medium containing 10% fetal bovine serum (hereinafter may be abbreviated as "serum-containing DMEM medium") was 346 µm.

In addition, the surface area of the hydrogel in a swollen state per 1 g of the hydrogel (1) in a dry state calculated from the average particle diameter using the following calculation method was 1,455 $cm^2$.

<Method for Calculating Surface Area per 1 g of Hydrogel in Dry State>

First, the surface area of one hydrogel in a swollen state was calculated by the following Equation (4) and the mass of one hydrogel in a dry state was calculated by the following Equation (5) using the average particle diameter of the hydrogel in a swollen state with phosphate-buffered saline. At this time, the particle diameter distribution was not taken into consideration, and the calculation was performed assuming a true sphere. The density of polyvinyl alcohol was 1.19 $g/cm^3$.

Surface area of one hydrogel in swollen state [$cm^2$/piece] = 4 x π x (Average particle diameter [cm]/2 of hydrogel in swollen state)$^2$     Equation (4)

Mass of one hydrogel in dry state [g/piece] = [4/3 x π x (Average particle diameter [cm]/2 of hydrogel in swollen state)$^3$] x (100/Degree of swelling [%]) x density of polyvinyl alcohol [$g/cm^3$]     Equation (5)

Next, the surface area per 1 g of the hydrogel in a dry state was calculated by the following Equation (6).

Surface area of hydrogel in swollen state per 1 g of hydrogel in dry state [$cm^2$/g] = Surface area of one hydrogel in swollen state [$cm^2$/piece]/Mass of one hydrogel in dry state [g/piece]     Equation (6)

<Cell Detachment by Suction and Discharge of at Least Part of Composition through Tube (Micropipette) Installed in Culture Vessel, and Separation Using Porous Material>

[Example 1]

The hydrogel (1) was dried overnight at normal temperature and under reduced pressure, and then 95 mg of the hydrogel was weighed. Since the surface area per 1 g of the hydrogel (1) in a dry state is 1,455 $cm^2/g$, the total surface area of the weighed hydrogel (1) is 138 $cm^2$.

Next, 30 mL of serum-containing DMEM medium was added to a sterilized Erlenmeyer flask (capacity: 125 mL, manufactured by Thermo Fisher Scientific) as a culture vessel, and the weighed hydrogel (1) was further added and allowed to stand for 30 minutes to swell. To the Erlenmeyer flask containing the swollen hydrogel (1), 8.10 x $10^5$ cultured mouse fibroblast NIH/3T3 cells (obtained from the cell bank ECACC) were added as raw material cells. The Erlenmeyer flask was placed on a shaker (manufactured by Yamato Scientific Co., Ltd.) installed in an incubator under a 5% $CO_2$ atmosphere at 37°C, and cultured at a rotation speed of 85 rpm for 4 days.

The obtained composition containing cells, hydrogel, and 30 mL of medium was transferred to a 50 mL centrifuge tube, and approximately 4.5 mL of the composition was sucked and discharged using a 5 mL micropipette (with a suction/discharge port diameter of 1 mm at the tip) to generate a liquid flow, thereby detaching the cultured cells from the surface of the hydrogel. One suction and one discharge were performed within approximately one second, and one set of one suction and one discharge was repeated 50 times. During suction and discharge, the micropipette was installed such that the micropipette suction/discharge port was always below the liquid level of the composition. Next, the obtained cell suspension was passed through a cell strainer (manufactured by Falcon) equipped with a mesh having an opening size of 100 $\mu$m, thereby filtering and separating the hydrogel and the cell suspension. The number of cells contained in the filtrate (cell suspension) was measured using a hemocytometer, and this was defined as the "cell count detached by pipetting". Next, in order to recover the cells remaining on the surface of the hydrogel on the cell strainer, a conventional cell recovering method using trypsin (concentration: 2.5 mg/mL) and EDTA (concentration: 0.29 mg/mL) was performed to detach the cells. Specifically, the hydrogel on the cell strainer was immersed in 5 mL of trypsin-EDTA solution, allowed to stand for 3 minutes in a 5% $CO_2$ atmosphere at 37 °C in an incubator, and then 5 mL of serum-containing DMEM medium was added to inactivate the trypsin. Thereafter, as in the above procedure, the hydrogel and cell suspension were separated by filtration using a cell strainer, and the number of cells contained in the obtained filtrate (cell suspension) was measured. This was defined as the "residual cell count detached by trypsin". In the subsequent microscopic observation, no cells were observed on the surface of the hydrogel after cell detachment by trypsin-EDTA. The cell recovery rate was calculated by the following Equation (7).

Cell recovery rate [%] = (Cell count detached by pipetting)/[(Cell count detached by pipetting + Residual cell count detached by trypsin)] x 100     Equation (7)

The same procedure was performed three times to determine the cell count detached by pipetting, residual cell count detached by trypsin, and the cell recovery rate. The average values of each were calculated and defined as the cell count detached by pipetting, the residual cell count detached by trypsin, and the cell recovery rate for hydrogel (1), respectively. The average value is shown in Table 1.

[Comparative Example 1]

NIH/3T3 cells were cultured in the same manner as the preparation step and the culturing step of Example 1, except that a commercially available cell culture carrier, Cytodex™ 3 (manufactured by Cytiva, main component: crosslinked dextran), was used instead of hydrogel (1). The surface area of Cytodex™ 3 was 2,000 $cm^2/g$ from the catalog value, and was added to a culture vessel such that the total area was 138 $cm^2$.

The cell count detached by pipetting, the residual cell count detached by trypsin, and the cell recovery rate of Cytodex™ 3 were determined in the same manner as in Example 1 except that the composition including the cells, the cell culture carrier, and the medium obtained above was used. The results are shown in Table 1.

[Comparative Example 2]

NIH/3T3 cells were cultured in the same manner as the preparation step and the culturing step of Example 1, except that a commercially available cell culture carrier, Corning (registered trademark) $C_{ellBIND}$ surface microcarrier (manufactured by Corning, main component: polystyrene), was used instead of hydrogel (1). The surface area of Corning (registered trademark) $C_{ellBIND}$ surface microcarrier was 360 $cm^2/g$ from the catalog value, and was added to a culture vessel such that the total area was 138 $cm^2$.

The cell count detached by pipetting, the residual cell count detached by trypsin, and the cell recovery rate of Corning (registered trademark) $C_{ellBIND}$ surface microcarrier were determined in the same manner as in Example 1 except that the composition including the cells, the cell culture carrier, and the medium obtained above was used. The results are shown in Table 1.

[Table 1]

| Table 1: Cell recovery rate by pipetting detachment (Separation using porous material) | | | | |
|---|---|---|---|---|
| | Seeding number (cells) | Cell count detached by pipetting (cells) | Cell count detached by trypsin (cells) | Cell recovery rate by pipetting detachment (%) |
| Example 1 | $8.10\times10^5$ | $1.90\times10^6$ | $1.75\times10^6$ | 52.1 |
| Comparative Example 1 | $8.10\times10^5$ | $4.20\times10^5$ | $2.50\times10^6$ | 14.4 |
| Comparative Example 2 | $8.10\times10^5$ | $1.05\times10^6$ | $3.25\times10^6$ | 24.4 |

As is clear from the results of Example 1 and Comparative Examples 1 and 2, when using a hydrogel containing a crosslinked body of a vinyl alcohol-based polymer, the cell recovery rate by pipetting detachment is higher compared to the case of using other commercially available cell culture carriers, and 50% or more of the cells can be recovered. Therefore, according to the present invention, the cells can be detached from the cell culture carrier by the liquid flow, and the cells of which the activity is maintained to be good can be stably produced.

<Cell Detachment by Shaking Composition, and Separation Using Porous Material>

[Example 2]

The Erlenmeyer flask after culturing, obtained in the same manner as the preparation step and the culturing step of Example 1, was installed in a shaker and shaken at 125 rpm for 10 minutes. For the obtained cell suspension, the hydrogel and the cell suspension were separated by filtration with a cell strainer in the same manner as in Example 1, the number of cells contained in the obtained filtrate (cell suspension) was measured, and this was defined as the "cell count detached by shaking".

From the composition contained in another Erlenmeyer flask after culture obtained in the same manner as the preparation step and the culturing step of Example 1, the hydrogel having cells on the surface was filtered with a cell strainer. For the hydrogel with cells present on the surface that was filtered, cells were detached using trypsin-EDTA in the same manner as in Example 1, and the number of cells was measured. This was defined as the "cell count detached by trypsin". In the subsequent microscopic observation, no cells were observed on the surface of the hydrogel after cell detachment by trypsin-EDTA. The cell recovery rate was calculated by the following Equation (8).

Cell recovery rate [%] = [(Cell count detached by shaking)/(Cell count detached by trypsin)] x 100          Equation (8)

The same procedure was performed three times to determine the cell count detached by shaking, the cell count detached by trypsin, and the cell recovery rate. The average values of each were calculated and defined as the cell count detached by shaking, the cell count detached by trypsin, and the cell recovery rate for hydrogel (1), respectively. The average value is shown in Table 2.

[Comparative Examples 3 and 4]

The cell count detached by shaking, the cell count detached by trypsin, and the cell recovery rate were determined for each of Cytodex™ 3 (Comparative Example 3) and Corning (registered trademark) CellBIND surface microcarriers (Comparative Example 4) in the same manner as in Example 2, except that the compositions containing the cells, the cell culture carrier, and the medium obtained in the preparation step and the culturing step of Comparative Example 1 and the preparation step and the culturing step of Comparative Example 2, respectively, were used. The results are shown in Table 2.

[Table 2]

| Table 2: Cell recovery rate by shaking detachment (Separation using porous material) | | | | |
|---|---|---|---|---|
| | Seeding number (cells) | Cell count detached by shaking (cells) | Cell count detached by trypsin (cells) | Cell recovery rate by shaking detachment (%) |
| Example 2 | $8.10 \times 10^5$ | $3.75 \times 10^6$ | $3.72 \times 10^6$ | 101 |
| Comparative Example 3 | $8.10 \times 10^5$ | $6.00 \times 10^5$ | $3.70 \times 10^6$ | 16.2 |
| Comparative Example 4 | $8.10 \times 10^5$ | $1.05 \times 10^6$ | $4.20 \times 10^6$ | 25.0 |

<Cell Detachment by Shaking Composition, Separation Utilizing Difference in Sedimentation Velocity>

[Example 3]

The Erlenmeyer flask after culturing, obtained in the same manner as the preparation step and the culturing step of Example 1, was installed in a shaker and shaken at 125 rpm for 10 minutes. The obtained cell suspension was transferred to a 50 mL centrifuge tube, 25 mL of serum-containing DMEM medium was added thereto, and the centrifuge tube was rotated and stirred. Then, the hydrogel was settled by standing the centrifuge tube upright for 1 minute, and approximately 20 mL of the cell suspension was recovered from the liquid level of the centrifuge tube. The number of cells contained in the recovered cell suspension was measured in the same manner as in Example 1. The same procedure was performed three times to determine the cell count detached by shaking. The average value was obtained and defined as the cell count detached by shaking of hydrogel (1). The average value is shown in Table 3.

[Table 3]

| Table 3: Cell count detached by shaking (Separation Using Difference in Sedimentation Velocity) | | |
|---|---|---|
| | Seeding number (cells) | Cell count detached by shaking (cells) |
| Example 3 | $8.10 \times 10^5$ | $3.90 \times 10^6$ |

<Reuse of Hydrogel from which Cells were Detached by Shaking Composition, as Cell Culture Carrier>

[Example 4]

The hydrogel settled in Example 3 was observed with a microscope, and it was confirmed that the cells were completely detached. This hydrogel was washed twice with 40 mL of PBS, and then used again as a cell culture carrier to perform cell culture in the same manner as in the culturing step of Example 1. Cells were detached from the hydrogel by subjecting the hydrogel after culture to cell detachment with trypsin-EDTA in the same manner as in Example 2, and the number of recovered cells was measured to be $3.80 \times 10^6$. Since the number of cells equivalent to that obtained by the culture using the novel hydrogel in Example 2 was obtained, it was confirmed that the hydrogel recovered by the production method of the present invention can be reused as a cell culture carrier.

[Example 5]

(Cell Count Detached by Trypsin)

The hydrogel (1) was dried overnight at normal temperature and under reduced pressure, and then 95 mg of the hydrogel was weighed. Since the surface area per 1 g of the hydrogel (1) in a dry state is 1,455 $cm^2$/g, the total surface area of the weighed hydrogel (1) is 138 $cm^2$.

Next, 30 mL of serum-containing DMEM medium was added to a sterilized Erlenmeyer flask (capacity: 125 mL, manufactured by Thermo Fisher Scientific) as a culture vessel, and the weighed hydrogel (1) was further added and allowed to stand for 30 minutes to swell. To the Erlenmeyer flask containing the swollen hydrogel (1), $8.10 \times 10^5$ cultured mouse fibroblast NIH/3T3 cells (obtained from the cell bank ECACC) were added as raw material cells. The Erlenmeyer flask was placed on a shaker (manufactured by Yamato Scientific Co., Ltd.) installed in an incubator under a 5% $CO_2$ atmosphere at 37°C, and cultured at a rotation speed of 85 rpm for 4 days.

The hydrogel was filtered from the composition containing the obtained cells, hydrogel, and 30 mL of medium using a cell strainer (manufactured by Falcon) equipped with a mesh having an opening size of 100 $\mu$m. Next, with respect to the filtered hydrogel, the conventional cell recovering method using trypsin (concentration: 2.5 mg/mL) and EDTA (concentration: 0.29 mg/mL) was performed to detach the cells. Specifically, the filtered hydrogel was immersed in 5 mL of trypsin-EDTA solution, allowed to stand for 3 minutes in a 5% $CO_2$ atmosphere at 37 °C in an incubator, and then 5 mL of serum-containing DMEM medium was added to inactivate the trypsin. Thereafter, as in the above procedure, the hydrogel and cell suspension were separated by filtration using a cell strainer, and the number of cells contained in the obtained filtrate (cell suspension) was measured. This was defined as the "cell count detached by trypsin".

(Number of Detached Cells When Trypsin-EDTA Solution is Used)

From a separate cultured composition obtained in the same manner as in the preparation step and culturing step described above, cells and hydrogel were similarly filtered. The filtered hydrogel was subjected to cell detachment using a trypsin-EDTA solution having the same EDTA concentration as the trypsin-EDTA solution used in the above (Cell Count Detached by Trypsin) but a different trypsin concentration. Specifically, the filtered hydrogel was immersed in 5 mL of trypsin-EDTA solution, allowed to stand for 3 minutes in a 5% $CO_2$ atmosphere at 37 °C in an incubator, and then 5 mL of serum-containing DMEM medium was added to inactivate the trypsin. Thereafter, as in the above procedure, the hydrogel and cell suspension were separated by filtration using a cell strainer, and the number of cells contained in the obtained filtrate (cell suspension) was measured. This was defined as the "number of detached cells using trypsin-EDTA solution".
The trypsin concentration was adjusted by diluting a trypsin (concentration: 2.5 mg/mL)-EDTA (concentration: 0.29 mg/mL) solution using a separately prepared PBS solution of EDTA (concentration: 0.29 mg/mL).
In addition, an EDTA PBS solution (concentration: 0.29 mg/mL) containing no trypsin was used as a solution having a trypsin concentration of 0 mg/mL, cell detachment was performed in the same manner as described above, and the number of detached cells when the solution was used was determined.

(Cell Recovery Rate)

The percentage (cell recovery rate) of the number of detached cells obtained using each trypsin-EDTA solution with different trypsin concentrations, relative to the cell count detached by trypsin, was calculated using the following Equation (9).

Cell recovery rate [%] = [(Number of detached cells when using each trypsin-EDTA solution)/(Cell count detached by trypsin)] x 100      Equation (9)

The same procedure was performed three times to determine the number of detached cells when using each trypsin-EDTA solution, the cell count detached by trypsin, and the cell recovery rate. The average values of each were calculated and defined as the number of detached cells when using each trypsin-EDTA solution, the cell count detached by trypsin, and the cell recovery rate for hydrogel (1), respectively. The average value of the cell recovery rate is shown in Table 4.

[Comparative Example 5]

NIH/3T3 cells were cultured in the same manner as the preparation step and the culturing step of Example 5, except that a commercially available cell culture carrier, Cytodex™ 3 (manufactured by Cytiva, main component: crosslinked dextran), was used instead of hydrogel (1). The surface area of Cytodex™ 3 was 2,000 cm²/g from the catalog value, and was added to a culture vessel such that the total area was 138 cm².
The cell recovery rate using each trypsin-EDTA solution for Cytodex™ 3 was determined in the same manner as in Example 5 except that the composition including the cells, the cell culture carrier, and the medium obtained above was used. The results are shown in Table 4.

[Comparative Example 6]

NIH/3T3 cells were cultured in the same manner as the preparation step and the culturing step of Example 5, except that a commercially available cell culture carrier, Corning (registered trademark) C$_{ellBIND}$ surface microcarrier (manufactured by Corning, main component: polystyrene), was used instead of hydrogel (1). The surface area of Corning (registered trademark) CellBIND surface microcarrier was 360 cm²/g from the catalog value, and was added to a culture vessel such that the total area was 138 cm².
The cell recovery rate when each trypsin-EDTA solution of a Corning (registered trademark) CellBIND surface microcarrier was used was determined in the same manner as in Example 5 except that the composition containing the cells, the cell culture carrier, and the medium obtained above was used. The results are shown in Table 4.

[Table 4]

Table 4: Cell recovery rate when using each trypsin-EDTA PBS solution with different trypsin concentrations

| Trypsin Concentration | 0 mg/mL | 0.5 mg/mL | 1.0 mg/mL | 1.3 mg/mL |
|---|---|---|---|---|
| Example 5 | 106.3% | 92.6% | 94.4% | 102.0% |
| Comparative Example 5 | 6.2% | 53.3% | 72.2% | 94.7% |
| Comparative Example 6 | 44.0% | 50.2% | 55.4% | 60.0% |

As is apparent from the results of Example 5 and Comparative Examples 5 and 6, when a hydrogel containing a crosslinked body of a vinyl alcohol-based polymer is used, the cell recovery rate is high even without substantially using a proteolytic enzyme as compared with the case of using other commercially available cell culture carriers. Therefore, according to the present invention, cells can be detached from the cell culture carrier using the chelating agent-containing solution in which the concentration of the proteolytic enzyme is 1.5 mg/mL or less, and cells of which activity is maintained well can be stably produced.

[Example 6]

(Cell Count Detached by Trypsin)

Cells and a hydrogel were filtered from the composition after culture obtained in the same manner as the preparation step and the culturing step of Example 5, using a cell strainer (manufactured by Falcon) equipped with a mesh having an opening size of 100 $\mu$m. In the same manner as in Example 5, with respect to the filtered hydrogel, the conventional cell recovering method using trypsin (concentration: 2.5 mg/mL) and EDTA (concentration: 0.29 mg/mL) was performed to detach the cells, and the number of detached cells was measured. This was defined as the "cell count detached by trypsin". The cell count detached by trypsin was $3.72 \times 10^6$.

(Number of detached cells when using EDTA PBS solution)

From a separate cultured composition obtained in the same manner as in the preparation step and culturing step described above, cells and hydrogel were similarly filtered. With respect to the filtered hydrogel, a EDTA PBS solution (EDTA concentration: 0.29 mg/mL) containing no trypsin was used as a solution having a trypsin concentration of 0 mg/mL, cell detachment was performed in the same manner as described above, and the "number of detached cells when using the EDTA PBS solution" when using the solution was determined.

In addition, cells were similarly detached using a separately prepared EDTA PBS solution not containing trypsin (EDTA concentration: 0.15 mg/mL, 0.07 mg/mL or 0.04 mg/mL), and the "number of detached cells when using the EDTA PBS solution" when using each solution was determined.

(Cell Recovery Rate)

The percentage (cell recovery rate) of the number of detached cells obtained using each EDTA PBS solution with different EDTA concentrations, relative to the cell count detached by trypsin, was calculated using the following Equation (10).

Cell recovery rate [%] = [(Number of detached cells when using each EDTA PBS solution)/(Cell count detached by trypsin)] x 100          Equation (10)

The same procedure was performed three times to determine the number of detached cells when using each EDTA PBS solution, the cell count detached by trypsin, and the cell recovery rate. The average values of each were calculated and defined as the number of detached cells when using each EDTA PBS solution, the cell count detached by trypsin, and the cell recovery rate for hydrogel (1), respectively. The average value of the cell recovery rate is shown in Table 5.

[Comparative Examples 7 and 8]

the cell recovery rate when using each EDTA PBS solution was determined for each of Cytodex™ 3 (Comparative Example 7) and Corning (registered trademark) C$_{ellBIND}$ surface microcarriers (Comparative Example 8) in the same manner as in Example 6, except that the compositions containing the cells, the cell culture carrier, and the medium obtained in the preparation step and the culturing step of Comparative Example 5 and the preparation step and the culturing step of Comparative Example 6, respectively, were used. The results are shown in Table 5.

[Table 5]

| Table 5: Cell recovery rate when using each EDTA PBS solution with different EDTA concentrations | | | | |
|---|---|---|---|---|
| EDTA concentration | 0.04 mg/mL | 0.07 mg/mL | 0.15 mg/mL | 0.29 mg/mL |
| Example 6 | 31.2% | 56.2% | 84.4% | 105.6% |
| Comparative Example 7 | 0.0% | 0.0% | 0.0% | 6.2% |
| Comparative Example 8 | 0.0% | 5.0% | 20.2% | 44.0% |

As is apparent from the results of Example 6 and Comparative Examples 7 and 8, when a hydrogel containing a crosslinked body of a vinyl alcohol-based polymer is used, the cell recovery rate is high as compared with the case of using other commercially available cell culture carriers. Therefore, according to the present invention, cells can be detached from the cell culture carrier even when using a chelating agent-containing solution that does not contain a proteolytic enzyme, and cells of which activity is maintained well can be stably produced.

[Example 7]

Cell detachment was performed in the same manner as in Example 6 except that the chelating agent in the EDTA PBS solution containing no trypsin was changed from EDTA (concentration: 0.29 mg/mL) to EGTA (concentration: 0.38 mg/mL) and the standing time was changed from 3 minutes to 5 minutes. As a result, the obtained cell recovery rate was 89.0%. From this result, it was confirmed that a similar cell detachment effect was obtained even when the chelating agent was changed from EDTA to EGTA.

[Example 8]

The hydrogel from which the cells were detached by a PBS solution of EDTA (0.29 mg/mL) in Example 6 was microscopically observed, and it was confirmed that the cells were completely detached. This hydrogel was washed twice with 40 mL of PBS, and then used again as a cell culture carrier to perform cell culture in the same manner as in the culturing step of Example 5. Cells were detached from the hydrogel by subjecting the hydrogel after culture to cell detachment with trypsin (concentration: 2.5 mg/mL) and EDTA (concentration: 0.29 mg/mL) by the same method as the operation used for measuring the cell count detached by trypsin in Example 6, and the number of recovered cells was measured to be 3.80 x $10^6$. Since the number of cells equivalent to that obtained by the culture using the novel hydrogel in Example 6 was obtained, it was confirmed that the hydrogel recovered by the production method of the present invention can be reused as a cell culture carrier.

**Claims**

1. A method for producing cells, comprising:

    a step of preparing a culture vessel comprising a composition comprising a liquid medium, a hydrogel containing a crosslinked body of a vinyl alcohol-based polymer, and raw material cells;
    a step of culturing the raw material cells on a surface of the hydrogel; and
    a step of detaching the cultured cells from the surface of the hydrogel by a liquid flow.

2. A method for producing cells, comprising:

    a step of preparing a culture vessel comprising a composition comprising a liquid medium, a hydrogel containing a crosslinked body of a vinyl alcohol-based polymer, and raw material cells;

a step of culturing the raw material cells on a surface of the hydrogel; and

a step of detaching the cultured cells from the surface of the hydrogel by bringing at least a part of the composition in the culture vessel into contact with a solution in which a concentration of a proteolytic enzyme is 1.5 mg/mL or less and a concentration of a chelating agent is 0.01 mg/mL or more.

3. The method according to claim 2, further comprising:
a step of detaching the cultured cells from the surface of the hydrogel by a liquid flow.

4. The method according to claim 1 or 3, wherein
the liquid flow is generated by one or more means selected from the group consisting of the following (i) to (iv):

(i) suction and discharge of at least a part of the composition through a tube installed in the culture vessel;
(ii) shaking of the composition;
(iii) stirring of the composition by stirring blades; and
(iv) discharge of a gas and/or a liquid into the culture vessel.

5. The method according to claim 1 or 2, wherein the hydrogel comprises a cell adhesion factor.

6. The method according to claim 1 or 2, wherein the hydrogel is non-spherical particles, spherical particles, a fine molded article, an article of any shape formed with a 3D printer, a film, filament, hollow fibers, a porous monolith, a coated article, or a mixture of two or more thereof.

7. The method according to claim 6, wherein the hydrogel in an equilibrium swollen state in a liquid medium is spherical particles having an average particle diameter of 10 to 5,000 $\mu$m.

8. The method according to claim 1 or 2, wherein the vinyl alcohol-based polymer has an ethylenically unsaturated group and has a viscosity-average degree of polymerization of 300 or more as measured in accordance with JIS K 6726:1994.

9. The method according to claim 2, wherein the chelating agent is ethylenediaminetetraacetic acid or ethylene glycol tetraacetic acid.

10. The method according to claim 1 or 2, wherein the culture vessel is a bioreactor.

11. The method according to claim 1 or 2, further comprising:
a step of separating the detached cells and the hydrogel.

12. The method according to claim 11, wherein the detached cells and the hydrogel are separated using a porous material.

13. The method according to claim 11, wherein the detached cells and the hydrogel are separated based on a difference in sedimentation velocity.

14. A method for producing cells, comprising:

a step of recovering the hydrogel separated by the method according to claim 11;
a step of preparing a culture vessel comprising a composition comprising a liquid medium, the recovered hydrogel, and raw material cells; and
a step of culturing the raw material cells on a surface of the recovered hydrogel.

## INTERNATIONAL SEARCH REPORT

| International application No. |
|---|
| **PCT/JP2024/017570** |

**A. CLASSIFICATION OF SUBJECT MATTER**

*C12N 5/071*(2010.01)i; *C12M 3/00*(2006.01)i; *C12N 5/07*(2010.01)i
FI: C12N5/071; C12M3/00 A; C12N5/07

According to International Patent Classification (IPC) or to both national classification and IPC

**B. FIELDS SEARCHED**

Minimum documentation searched (classification system followed by classification symbols)

C12N5/071; C12M3/00; C12N5/07

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2006-246883 A (FUJI PHOTO FILM CO., LTD.) 21 September 2006 (2006-09-21) claims, paragraphs [0044], [0089]-[0098] | 1-14 |
| X | JP 2010-158180 A (HOKKAIDO UNIV.) 22 July 2010 (2010-07-22) claims, paragraphs [0047], [0053], [0073]-[0079] | 1-14 |
| A | WO 2017/104618 A1 (NIPPON ZEON CO.) 22 June 2017 (2017-06-22) entire text | 1-14 |

☐ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 July 2024** | **16 July 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2024/017570**

| Patent document cited in search report | | | Publication date (day/month/year) | Patent family member(s) | | | Publication date (day/month/year) |
|---|---|---|---|---|---|---|---|
| JP | 2006-246883 | A | 21 September 2006 | (Family: none) | | | |
| JP | 2010-158180 | A | 22 July 2010 | (Family: none) | | | |
| WO | 2017/104618 | A1 | 22 June 2017 | US | 2018/0355309 | A1 | |
| | | | | EP | 3392335 | A1 | |
| | | | | CN | 108368480 | A | |
| | | | | KR | 10-2018-0088858 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023080024 A
- JP 2023080030 A
- JP 2022536651 A
- JP 11349643 A
- WO 2017104618 A1
- JP 2018201403 A

**Non-patent literature cited in the description**

- *Biotechnology and Bioengineering*, 2012, vol. 109, 1561-1570
- *Journal of Membrane Science*, 2017, vol. 524, 79-86
- *Chemical Engineering and Technology*, 2008, vol. 31, 1099-1115
- *Chemical Papers*, 2008, vol. 62, 364-374